# EUROPEAN PATENT SPECIFICATION

(11) **EP 2 585 833 B1**
(45) Date of publication and mention of the grant of the patent: **31.05.2017**
(21) Application number: 11798823.8
(22) Date of filing: 22.06.2011
(51) Int. Cl.: G01N 33/92, G01N 33/50

(54) **METHODS FOR DETECTING CANCER**
VERFAHREN ZUR KREBSERKENNUNG
PROCÉDÉS DE DÉTECTION DE CANCER

(30) Priority: 23.06.2010 US 357642 P
(43) Date of publication of application: 01.05.2013
(60) Divisional application: 17163073.4
(73) Proprietor: University of Louisville Research Foundation, Inc., Louisville, KY 40202-1959 (US)
(72) Inventor: FAN, Teresa, Whei-mei, Lexington, KY 40509 (US); LANE, Andrew, Nicholas, Lexington, KY 40509 (US); HIGASHI, Richard, M., Lexington, KY 40513 (US); BOUSAMRA, Michael, Louisville, KY 40241 (US)
(74) Representative: Jacob, Reuben Ellis
(86) International application number: PCT/US2011/041399
(87) International publication number: WO 2011/163332

(56) References cited:
- WO-A1-2009/096903
- DESSI S ET AL: "Altered pattern of lipid metabolism in patients with lung cancer", ONCOLOGY, S. KARGER, BASEL, CH, vol. 49, no. 6, 1 January 1992 (1992-01-01), pages 436-441, XP009087393, ISSN: 0030-2414
- FRANKY DHAVAL SHAH ET AL: "Significance of alterations in plasma lipid profile levels in breast cancer.", INTEGRATIVE CANCER THERAPIES MAR 2008, vol. 7, no. 1, March 2008 (2008-03), pages 33-41, XP008165142, ISSN: 1534-7354
- NIRAJ KUMAR SRIVASTAVA ET AL: "In vitro , high-resolution 1 H and 31 P NMR based analysis of the lipid components in the tissue, serum, and CSF of the patients with primary brain tumors: one possible diagnostic view", NMR IN BIOMEDICINE, 1 January 2009 (2009-01-01), pages n/a-n/a, XP55077655, ISSN: 0952-3480, DOI: 10.1002/nbm.1427
- QADIR M I ET AL: "Plasma lipid profile in gynecologic cancers.", EUROPEAN JOURNAL OF GYNAECOLOGICAL ONCOLOGY 2008, vol. 29, no. 2, 2008, pages 158-161, XP55077657, ISSN: 0392-2936
- HALTON J M ET AL: "Blood lipid profiles in children with acute lymphoblastic leukemia", CANCER 19980715 US, vol. 83, no. 2, 15 July 1998 (1998-07-15), pages 379-384, XP55077665, ISSN: 0008-543X

## Description

### BACKGROUND

Cancer is a major cause of death and suffering and is a major cost to medical systems in the U.S. and across the world. Various studies have been carried out in an attempt to increase understanding in this area. Dessi et al (Oncology, S. Karger, Basel, CH, Vol. 49, No. 6, 1 January 1992, pages 436-441) refers to studies showing an altered pattern of lipid metabolism in patients with lung cancer. Shah et al (Integrative Cancer Therapies, Vol. 7, No. 1, March 2008, pages 33-41) relates to alterations in plasma lipid profiles in breast cancer. Srivastava et al (NMR in Biomedicine, 1 January 2009, pages 113-122) relates to in vitro, high resolution 1H and 31P NMR based analysis of the lipid components in the tissue, serum and cerebrospinal fluid of patients with primary brain tumours. Qadir et al (European Journal of Gynaecological Oncology, Vol. 29, No. 2, 2008, pages 158-161) relates to plasma lipid profile in gynaelogical cancers. Halton et al (1998 American Cancer Society, Vol. 83, No. 2, 15 July 1998, pages 379-384) relates to blood lipid profiles in children with acute lymphoblastic leukemia. WO2009/096903 relates to a method of generating a classification model capable of distinguishing between two biological states; such as a normal and a cancerous or tumour state. Early detection of cancer is associated with better treatment options and improved outcome. Therefore, early detection of cancer can help minimize both the suffering and the cost, while typically increasing the chance of survival. Thus, some embodiments of the invention are methods to determine the presence of cancers in animals.

### SUMMARY

According to a first aspect, the invention relates to a method for determining the presence or absence of at least one cancer type in an animal comprising
- determining lipids amounts of lipids in a lipid set in a sample from the animal, and
- determining the presence or absence of at least one cancer type in the animal with a predictive model;
   wherein
   - the lipid amounts of lipids in the lipid set comprise an input of the predictive model,
   - the sample comprises a bodily fluid or treatment thereof;
   - the at least one cancer type is selected from the group consisting of carcinomas, sarcomas, hematologic cancers, neurological malignancies, thyroid cancer, neuroblastoma, melanoma, renal cell carcinoma, hepatocellular carcinoma, breast cancer, colon cancer, lung cancer, pancreatic cancer, brain cancer, prostate cancer, chronic lymphocytic leukemia, acute lymphoblastic leukemia, rhabdomyosarcoma, Glioblastoma multiforme, meningioma, bladder cancer, gastric cancer, Glioma, oral cancer, nasopharyngeal carcinoma, kidney cancer, rectal cancer, lymph node cancer, bone marrow cancer, stomach cancer, uterine cancer, leukemia, basal cell carcinoma, cancers related to epithelial cells, cancers that can alter the regulation or activity of Pyruvate Carboxylase, and tumors associated with any of the aforementioned cancer types;
   - the lipid set comprises a lipid from the class of BMP, preferably BMP (30:1), BMP (32:1), BMP (34:1), BMP (35:4), BMP (36:3), BMP (37:1), BMP (37:7), BMP (38:1), BMP (38:2), BMP (38:4), BMP (39:1), BMP (39:4), BMP (40:1), BMP (40:2), BMP (40:3), BMP (40:4), BMP (40:7), BMP (42:10), BMP (42:2), BMP (42:5), or BMP (44:8), and
   - the predictive model comprises one or more of dimension reduction method, clustering method, machine learning method, principle component analysis, soft independent modeling of class analogy, partial least squares regression, orthogonal least squares regression, partial least squares discriminant analysis, orthogonal partial least squares discriminant analysis, mean centering, median centering, Pareto scaling, unit variance scaling, orthogonal signal correction, integration, differentiation, cross validation, or receiver operating characteristic curves.

Preferably, the bodily fluid is selected from the group consisting of plasma vomit, cerumen, gastric juice, breast milk, mucus, saliva, sebum, semen, sweat, tears, vaginal secretion, blood serum, aqueous humor, vitreous humor, endolymph, perilymph, peritoneal fluid, pleural fluid, cerebrospinal fluid, blood, plasma, nipple aspirate fluid, urine, stool, and bronchioalveolar lavage fluid.

Preferably, the sample comprises a lipid microvesicle fraction.

Preferably, the lipid set comprises at least 10, at least 50, at least 100, at least 200 or no more than 100,000 lipids.

Preferably, the lipid set further comprises one or more lipids selected from the one or more classes of lipids selected from the group consisting of CE, Cer, DAG, DH-LTB4, FA, GA2, GM3, HexCer, HexDHCer, LacCer, LysoPA, LysoPC, LysoPC-pmg, LysoPE, LysoPE-pmg, LysoPS, MAG, PC, PC-pmg, PE, PE-pmg, PGA1, PGB1, SM, Sphingosine, TAG, and TH-12-keto-LTB4.

Preferably, the lipid set further comprises one or more lipids selected from the one or more classes of lipids selected from the group consisting of FA, MAG, DAG, TAG, PI, PE, PS, PI, PG, PA, LysoPC, LysoPE, LysoPS, LysoPI, LysoPG, LysoPA, LysoPC, LysoPE, SM, Cer, Cer-P, HexCer, GA1, GA2, GD1, GD2, GM1, GM2, GM3, GT1, and CE.

Preferably, one or more lipids in the lipid set are further selected from the group consisting of CE (16:2), CE (18:2), CE (18:3), CE (18:4), CE (20:2), CE (20:4), CE (20:5), Cer (32:1), Cer (34:1), Cer (36:1), Cer (38:1), Cer (38:4), Cer (40:2), Cer (40:4), DAG (28:0), DAG (32:0), DAG (32:2), DAG (34:0), DAG (34:3), DAG (34:5), DAG (36:0), DAG (36:1), DAG (36:2), DAG (36:3), DAG (36:8), DAG (38:1), DAG (38:10), DAG (38:2), DAG (38:3), DAG (38:5), DAG (40:1), DAG (40:2), DAG (40:5), DH-LTB4 (20:3), FA (16:3), FA (19:1), GA2 (30:0), GA2 (33:2), GA2 (35:2), GA2 (37:2), GM3 (41:1), HexCer (32:1), HexDHCer (34:0), LacCer (30:0), LacCer (30:1), LacCer (32:2), LysoPA (16:2), LysoPA (16:3), LysoPA (18:1), LysoPA (22:0), LysoPA (22:1), LysoPC (16:0), LysoPC (18:0), LysoPC (18:1), LysoPC (18:4), LysoPC (20:4), LysoPC (20:5), LysoPC (26:6), LysoPC-pmg (12:0), LysoPC-pmg (18:3), LysoPC-pmg (24:4), LysoPC-pmg (26:0), LysoPE (10:1), LysoPE (16:2), LysoPE (18:2), LysoPE-pmg (18:4), LysoPS (24:1), MAG (18:0), MAG (20:3), MAG (24:2), PC (32:0), PC (32:1), PC (34:1), PC (34:1), PC (34:2), PC (34:3), PC (34:4), PC (34:6), PC (36:1), PC (36:2), PC (36:3), PC (36:4), PC (36:5), PC (36:6), PC (36:9), PC (38:2), PC (38:3), PC (38:4), PC (38:5), PC (38:6), PC (38:7), PC (38:8), PC (38:9), PC (40:5), PC (40:6), PC (40:7), PC (40:8), PC (40:9), PC (44:12), PC-pmg (30:1), PC-pmg (36:4), PC-pmg (38:5), PC-pmg (38:7), PC-pmg (40:11), PC-pmg (42:1), PE (34:7), PE (36:5), PE (36:7), PE (38:2), PE (38:3), PE (38:4), PE (38:5), PE (38:7), PE (40:4), PE (40:9), PE (42:12), PE (44:11), PE-pmg (28:2), PE-pmg (30:3), PE-pmg (34:6), PE-pmg (34:8), PE-pmg (36:5), PE-pmg (36:6), PE-pmg (40:7), PE-pmg (40:8), PE-pmg (42:10), PE-pmg (42:12), PE-pmg (42:4), PE-pmg (42:7), PE-pmg (42:8), PE-pmg (42:9), PE-pmg (44:10), PE-pmg (44:11), PE-pmg (44:12), PE-pmg (44:7), PE-pmg (44:8), PE-pmg (44:9), PGA1 (20:1), PGB1 (20:1), SM (34:1), SM (34:2), SM (36:1), SM (38:1), SM (40:1), SM (40:2), SM (42:1), SM (42:2), SM (42:3), Sphingosine (18:0), TAG (44:1), TAG (44:3), TAG (46:0), TAG (46:1), TAG (46:2), TAG (46:3), TAG (46:4), TAG (48:0), TAG (48:1), TAG (48:2), TAG (48:3), TAG (48:4), TAG (48:5), TAG (49:1), TAG (49:2), TAG (49:3), TAG (50:0), TAG (50:1), TAG (50:2), TAG (50:3), TAG (50:4), TAG (50:5), TAG (50:6), TAG (51:2), TAG (51:4), TAG (52:2), TAG (52:3), TAG (52:4), TAG (52:5), TAG (52:6), TAG (52:7), TAG (53:4), TAG (54:2), TAG (54:3), TAG (54:4), TAG (54:5), TAG (54:6), TAG (54:7), TAG (54:8), TAG (55:5), TAG (55:6), TAG (55:7), TAG (56:4), TAG (56:5), TAG (56:6), TAG (56:7), TAG (56:8), TAG (56:9), TAG (58:10), TAG (58:6), TAG (58:8), TAG (58:9), TAG (60:12), and TH-12-keto-LTB4(20:2).

Preferably, the at least one cancer type comprises lung cancer and one or more lipids in the lipid set are further selected from the group consisting of LysoPA (22:0), PE-pmg (42:9), FA (16:3), FA (19:1), CE (18:2), Cer (36:1), Cer (38:4), PC (38:5), Cer (38:1), and TAG (44:3).

Preferably, the at least one cancer type comprises lung cancer and one or more lipids in the lipid set are further selected from the group consisting of TAG (44:3), PC (36:5), PC (38:5), Cer (38:4), PE-pmg (42:9), PC (38:7), LysoPA (22:0), Cer (38:1), Cer (34:1), Cer (36:1), PC (40:7), TAG (54:5), TAG (54:6), CE (18:2), PC (36:4), FA (16:3), PE-pmg (44:11), TAG (52:5), Cer (40:4), CE (20:5), PC (38:6), TAG (50:2), MAG (18:0), FA (19:1), TAG (52:2), LysoPA (22:1), MAG (24:2), TAG (54:7), TAG (50:3), TAG (50:1), DAG (36:3), PC (34:1), TAG (52:6), PE-pmg (44:12), CE (20:4), PE (44:11), PC (40:8), TAG (56:9), PE-pmg (34:6), PE (36:7), PE (36:5), TAG (56:7), TAG (56:8), DAG (34:3), TAG (56:6), TAG (52:3), TAG (54:3), TAG (56:5), TAG (54:8), PC (34:6), PC (40:6), DAG (36:0), LysoPE (10:1), DAG (40:5), Cer (32:1), TAG (50:5), TAG (50:4), PE-pmg (36:6), TAG (46:3), and PE (38:5).

Preferably, the at least one cancer type comprises lung cancer and one or more lipids in the lipid set are further selected from the group consisting of TAG (44:3), PC (36:5), PC (38:5), Cer (38:4), PE-pmg (42:9), PC (38:7), LysoPA (22:0), Cer (38:1), Cer (34:1), and Cer (36:1).

Preferably, the at least one cancer type comprises breast cancer and one or more lipids in the lipid set are further selected from the group consisting of LysoPA (22:1), PE-pmg (42:9), CE (20:5), TAG (52:3), LysoPA (22:0), PC (36:3), PC (36:4), PC (36:2), PC (34:2), and PC (34:1).

Preferably, the at least one cancer type comprises breast cancer and one or more lipids in the lipid set are further selected from the group consisting of PC (34:2), PC (34:1), PC (36:2), PC (36:4), PC (36:3), PC (38:4), LysoPA (22:1), PE-pmg (42:9), LysoPA (22:0), CE (20:5), Cer (36:1), CE (18:2), DAG (34:0), SM (34:1), DAG (32:0), PE-pmg (40:8), PC (38:3), DAG (36:0), PC (36:1), TAG (54:5), TAG (54:6), PE-pmg (44:11), PE-pmg (42:8), TAG (52:2), SM (42:2), PC (38:6), TAG (54:7), PC (40:6), PC (40:7), LysoPC (16:0), FA (16:3), TAG (52:5), TAG (44:3), SM (40:1), PE-pmg (42:10), PE-pmg (40:7), SM (36:1), PE (38:2), PC (34:3), PC (36:5), PC (32:0), PC (32:1), PC (36:9), SM (42:3), PC-pmg (36:4), PC-pmg (38:5), PC (40:9), TAG (54:3), PE-pmg (44:12), FA (19:1), TAG (50:3), PC (34:6), GA2 (35:2), TAG (58:9), PE-pmg (42:7), and LysoPC (18:0).

Preferably, the at least one cancer type comprises breast cancer and one or more lipids in the lipid set are further selected from the group consisting of PC (34:2), PC (34:1), PC (36:2), PC (36:4), PC (36:3), PC (38:4), LysoPA (22:1), PE-pmg (42:9), LysoPA (22:0), and CE (20:5).

Preferably, the at least one cancer type comprises lung cancer and breast cancer, and one or more lipids in the lipid set are further selected from the group consisting of LysoPA (22:1), PC (36:5), TAG (52:3), PC (38:5), CE (20:5), TAG (50:2), PC (34:2), CE (18:2), and PC (34:1).

Preferably, the at least one cancer type comprises lung cancer and breast cancer, and one or more lipids in the lipid set are further selected from the group consisting of PC (34:2), PC (36:2), TAG (44:3), CE (18:2), PC (34:1), LysoPA (22:1), PC (36:5), Cer (36:1), CE (20:5), PC (36:3), PC (38:4), PC (36:4), Cer (38:4), PC (38:5), PC (38:7), Cer (38:1), TAG (50:2), Cer (34:1), SM (34:1), Cer (40:4), MAG (18:0), MAG (24:2), PC (38:3), PE-pmg (40:8), PE-pmg (42:8), TAG (50:1), DAG (32:0), PC (36:1), DAG (34:0), LysoPC (16:0), PE-pmg (34:6), DAG (36:3), PC (36:9), PE (36:5), TAG (52:6), FA (19:1), PE-pmg (44:11), PE (44:11), TAG (48:2), SM (42:2), PE-pmg (42:10), PE (36:7), PE-pmg (40:7), PE-pmg (36:6), PE (38:5), PC (32:0), PE (38:2), GA2 (35:2), DAG (34:3), PE-pmg (44:12), MAG (16:0), PC (32:1), LysoPE (10:1), SM (36:1), TAG (56:7), and PE-pmg (42:9).

Preferably, the at least one cancer type comprises lung cancer and breast cancer, and one or more lipids in the lipid set are further selected from the group consisting of PC (34:2), PC (36:2), TAG (44:3), CE (18:2), PC (34:1), LysoPA (22:1), PC (36:5), Cer (36:1), CE (20:5), and PC (36:3).

Preferably, the lipid amounts are determined using mass spectrometry, such as a Fourier transform ion cyclotron resonance mass analyzer.

Preferably, the sample is a treatment of a bodily fluid and the treatment comprises one or more extractions using one or more solutions comprising acetonitrile, water, chloroform, methanol, butylated hydroxytoluene, trichloroacetic acid, or combinations thereof.

Preferably, the predictive model comprises one or more dimension reduction methods.

Preferably, the predictive model comprises one or more methods selected from the group consisting of principal component analysis (PCA), soft independent modeling of class analogy (SIMCA), partial least squares discriminant analysis (PLS-DA), and orthogonal partial least squares discriminant analysis (OPLS-DA).

Preferably, the animal is selected from the group consisting of human, dog, cat, horse, cow, pig, sheep, chicken, turkey, mouse, and rat.

Preferably, the at least one cancer type is lung or breast cancer.

### BRIEF DESCRIPTION OF THE DRAWINGS

The following drawings form part of the present specification and are included to further demonstrate certain aspects of the present invention. The invention may be better understood by reference to one or more of these drawings in combination with the description of specific embodiments presented herein.
Figure 1. Diacylglycerols in samples from humans with lung cancer, breast cancer, and no cancer (control).
Figure 2. Phosphatidylcholines in samples from humans with lung cancer, breast cancer, and no cancer (control).
Figure 3. Phosphatidylcholines in samples from humans with lung cancer, breast cancer, and no cancer (control).
Figure 4. Phosphatidylcholines in samples from humans with lung cancer, breast cancer, and no cancer (control).
Figure 5. Phosphatidylcholines in samples from humans with lung cancer, breast cancer, and no cancer (control).
Figure 6. Phosphatidylethanolamines in samples from humans with lung cancer, breast cancer, and no cancer (control).
Figure 7. Phosphatidylethanolamines in samples from humans with lung cancer, breast cancer, and no cancer (control).
Figure 8. Phosphatidylethanolamines in samples from humans with lung cancer, breast cancer, and no cancer (control).
Figure 9. Phosphatidylethanolamines-pmg in samples from humans with lung cancer, breast cancer, and no cancer (control).
Figure 10. Phosphatidylethanolamines-pmg in samples from humans with lung cancer, breast cancer, and no cancer (control).
Figure 11. Triacylglycerols in samples from humans with lung cancer, breast cancer, and no cancer (control).
Figure 12. Monoacylglycerols in samples from humans with lung cancer, breast cancer, and no cancer (control).
Figure 13. Lipid classes in samples from humans comparing cancer type.
Figure 14. PC1-PC3 PCA scores plot for breast cancer, control, and lung cancer samples - 4 components, R²X = 0.475, Q² = 0.296.
Figure 15. PC1-PC3 PCA loadings plot for breast cancer, control, and lung cancer samples - 4 components, R²X = 0.475, Q² = 0.296.
Figure 16. OPLS-DA scores plot separating lung cancer and control samples - 1+1 orthogonal components, R²X = 0.253, R²Y = 0.619, Q² = 0.345.
Figure 17. OPLS-DA coefficients plot separating lung cancer and control samples - 1+1 orthogonal components, R²X = 0.253, R²Y = 0.619, Q² = 0.345. The coefficients plot indicates lipids elevated in control samples.
Figure 18. OPLS-DA coefficients plot separating lung cancer and control samples - 1+1 orthogonal components, R²X = 0.253, R²Y = 0.619, Q² = 0.345. The coefficients plot indicates lipids elevated in lung cancer samples.
Figure 19. OPLS-DA scores plot separating breast cancer and control samples - 1+1 orthogonal components, R²X = 0.281, R²Y = 0.762, Q² = 0.625.
Figure 20. OPLS-DA coefficients plot separating breast cancer and control samples - 1+1 orthogonal components, R²X = 0.281, R²Y = 0.762, Q² = 0.625. The coefficients plot indicates lipids elevated in control samples.
Figure 21. OPLS-DA coefficients plot separating breast cancer and control samples - 1+1 orthogonal components, R²X = 0.281, R²Y = 0.762, Q² = 0.625. The coefficients plot indicates lipids elevated in breast cancer samples.
Figure 22. OPLS-DA scores plot separating lung cancer and breast cancer samples - 1+1 orthogonal components, R²X = 0.309, R²Y = 0.816, Q² = 0.725.
Figure 23. OPLS-DA coefficients plot separating lung cancer and breast cancer samples - 1+1 orthogonal components, R²X = 0.309, R²Y = 0.816, Q² = 0.725. The coefficients plot indicates lipids elevated in lung cancer samples.
Figure 24. OPLS-DA coefficients plot separating lung cancer and breast cancer samples - 1+1 orthogonal components, R²X = 0.309, R²Y = 0.816, Q² = 0.725. The coefficients plot indicates lipids elevated in breast cancer samples.
Figure 25. A flow chart representing an embodiment of one of the methods disclosed herein. Cancer status can include the presence or absence of one or more cancer types.

### DETAILED DESCRIPTION

Some embodiments of the invention include methods for detecting the presence or absence of one or more cancer types by determining the amount of lipids in as lipid set in a sample. The sample can be a bodily fluid (or treatment thereof) from an animal. In some instances, the sample (e.g., a bodily fluid extract) comprises a concentration of lipid microvesicles that is higher than normally found in a bodily fluid. The lipid amounts in the lipid set are analyzed using a predictive model to determine the presence or absence of one or more cancer types.

Lipids are designated according to the following notation XXX (YY:ZZ). XXX is the abbreviation for the lipid group (in many instances indicating the lipid headgroup) as provided, for example in Table 1. YY is the number of carbons in the acyl chain. ZZ is the number of double bonds in the acyl chains.

The term lipid, as used herein, is defined as a collection of one or more isomers. For example, PC (36:1) is a lipid and is the collection of one or more of the phosphatidylcholine isomers that have 36 carbons in the acyl chain and one double bond in either of the two acyl chains; these isomers have identical molecular weights. Although the term lipid can encompass the entire collection of isomers, the sample may, in fact, have only one isomer, several isomers, or any number of isomers less than the total number of all possible isomers in a collection. Accordingly, lipid can refer to one or more of the isomers that make up the entire collection of possible isomers.

The term lipid set is defined to include one or more lipids.

The term lipid amount (and similar phrases such as, amounts of lipids or amount of a lipid) is defined to encompass an absolute amount of a lipid (e.g., in mmoles) or a relative amount of a lipid (e.g., in % relative intensity).

Table 1 provides lipid name abbreviations used in the data; other abbreviations are provided in the text as needed.

**Table 1**

| **Abbreviation** | **Name** |
|---|---|
| BMP | Bis(monoacylglycero)phosphates |
| CE | Cholesterol Esters |
| Cer | Ceramides |
| DAG | Diacylglycerols |
| DH-LTB4 | Dihydroleukotriene b4 |
| FA | Fatty Acids |
| GA2 | Gangliosides A2 |
| GM3 | Gangliosides M3 |
| HexCer | Hexose Ceramides |
| HexDHCer | Hexosyl dihydroceramide |
| LacCer | Lactosylceramide |
| LysoPA | Lysophosphatidic acid |
| LysoPC | Lysophosphatidylcholines |
| LysoPC-pmg | Lysophosphatidylcholines-plasmalogens |
| LysoPE | Lysophosphatidylethanolamines |
| LysoPE-pmg | Lysophosphatidylethanolamines-plasmalogens |
| LysoPS | Lysophosphatidylserines |
| MAG | Monoacylglycerols |
| PC | Phospahtidylcholines |
| PC-pmg | Phosphatidylcholines-plasmalogens |
| PE | Phosphatidylethanolamines |
| PE-pmg | Phosphatidylethanolamines-plasmalogens |
| PGA1 | Prostaglandin A1 |
| PGB1 | Prostaglandin B1 |
| SM | Sphingomyelins |
| Sphingosine | Sphingosine |
| TAG | Triacylglycerols |
| TH-12-keto-LTB4 | Tetrahydro-12-keto-leukotriene b4 |

Bodily fluids can be any suitable bodily fluid for the determination of cancer and include but are not limited to vomit, cerumen (earwax), gastric juice, breast milk, mucus (e.g., nasal drainage and phlegm), saliva, sebum (skin oil), semen (including prostatic fluid), sweat, tears, vaginal secretion, blood serum, aqueous humor, vitreous humor, endolymph, perilymph, peritoneal fluid, pleural fluid, cerebrospinal fluid, blood, plasma, nipple aspirate fluid, urine, stool, bronchioalveolar lavage fluid, peripheral blood, sera, plasma, ascites, cerebrospinal fluid (CSF), sputum, bone marrow, synovial fluid, aqueous humor, amniotic fluid, Cowper's fluid or pre-ejaculatory fluid, female ejaculate, fecal matter, hair, cyst fluid, pleural and peritoneal fluid, pericardial fluid, lymph, chyme, chyle, bile, interstitial fluid, menses, pus, vaginal secretions, mucosal secretion, stool water, pancreatic juice, lavage fluids from sinus cavities, bronchopulmonary aspirates, or other lavage fluids.

The bodily fluid can originate from any animal tissue (e.g., mammalian tissues) including, but not limited to connective tissue, muscle tissue, nervous tissue, adipose tissue, endothelial tissue, or epithelial tissue. The tissue can be at least part of an organ or part of an organ system. Organs can include, but are not limited to heart, blood, blood vessels, salivary glands, esophagus, stomach, liver, gallbladder, pancreas, large intestines, small intestines, rectum, anus, colon, endocrine glands (e.g., hypothalamus, pituitary, pineal body, thyroid, parathyroids and adrenals), kidneys, ureters, bladder, urethraskin, hair, nails, lymph, lymph nodes, lymph vessels, leukocytes, tonsils, adenoids, thymus, spleen, muscles, brain, spinal cord, peripheral nerves, nerves, sex organs (e.g., ovaries, fallopian tubes, uterus, vagina, mammary glands (e.g., breasts), testes, vas deferens, seminal vesicles, prostate and penis), pharynx, larynx, trachea, bronchi, lungs, diaphragm, bones, cartilage, ligaments, or tendons. Organ systems can include, but are not limited to circulatory system, digestive system, endocrine system, excretory system, integumentary system, lymphatic system, muscular system, nervous system, reproductive system, respiratory system, or skeletal system.

Bodily fluids can be frozen in liquid nitrogen. Preparation of the removed bodily fluids can be performed in any suitable manner.

In some embodiments, the bodily fluid is the sample. In other embodiments, the bodily fluid is treated to provide the sample. Treatment can include any suitable method, including but not limited to extraction, centrifugation (e.g., ultracentrifugation), lyophilization, fractionation, separation (e.g., using column or gel chromatography), or evaporation. In some instances, this treatment can include one or more extractions with solutions comprising any suitable solvent or combinations of solvents, such as, but not limited to acetonitrile, water, chloroform, methanol, butylated hydroxytoluene, trichloroacetic acid, toluene, hexane, benzene, or combinations thereof. For instance, in some embodiments, fractions from blood are extracted with a mixture comprising methanol and butylated hydroxytoluene. In some instances, the sample (e.g., a bodily fluid extract or a lipid microvesicle fraction of blood plasma) comprises a concentration of lipid microvesicles that is higher than normally found in a bodily fluid.

The volume of the sample (e.g., the bodily fluid or treatment thereof) used for analyzing can be in the range of about 0.1 mL to about 20 mL, such as no more than about 20, about 15, about 10, about 9, about 8, about 7, about 6, about 5, about 4, about 3, about 2, about 1, or about 0.1 mL.

Broad classes of lipids that can be part of a lipid set include, but are not limited to, fatty acids (characterized by a carboxyl group and an acyl chain), Glycerolipids (characterized by the presence of a glycerol backbone with one - monoacylglycerols (MAG), two - diacylglycerols (DAG) or three - triacylglycerols (TAG) ester linked fatty acyl chains), glycerophospholipids (GPL) (characterized by a glyceryl backbone with two ester-linked acyl chains and a phosphate-linked polar headgroup - GPLs include phosphatidylcholines (PC), phosphatidylethanolamines (PE), phosphatidylserines (PS), phosphatidylglycerols (PG), phosphatidylinositols (PI), inositol, and phosphatidic acids), Lysoglycerophospholipids (LGPL) (LGPLs are missing one of the acyl chains at the glycerol backbone, e.g., at the C2 position - LGPLs include include lysophospahtidylcholines (LysoPC), lysophosphatidylethanolamines (LysoPE), lysophosphatidylserines (LysoPS), lysophosphatidylglycerols (LysoPG), lysophosphatidylinositols (LysoPI), lysoinositol, and lysophosphatidic acids), sphingolipids (SPL) (characterized by a sphingosine base backbone with a trans double bond between C4 and C5 of an acyl chain linked to the amino group via an amide linkage), Bis(monoacylglycero)phosphate (BMP), Ceramides, Gangliosides, Sterols, Prenols, Saccharolipids, and Polyketides. In some instances, the lipid group is based on the acyl chain composition which can vary in any number of ways including the number of carbons in the acyl chain and the number of double bonds in the acyl chain.

In other embodiments, lipids in the lipid set can come from one or more classes of lipids, such as, BMP, CE, Cer, DAG, DH-LTB4, FA, GA2, GM3, HexCer, HexDHCer, LacCer, LysoPA, LysoPC, LysoPC-pmg, LysoPE, LysoPE-pmg, LysoPS, MAG, PC, PC-pmg, PE, PE-pmg, PGA1, PGB1, SM, Sphingosine, TAG, or TH-12-keto-LTB4. In still other embodiments, lipids in the lipid set can come from one or more classes of lipids, such as, FA, MAG, DAG, TAG, PC, PE, PS, PI, PG, PA, LysoPC, LysoPE, LysoPS, LysoPI, LysoPG, LysoPA, LysoPC, LysoPE, BMP, SM, Cer, Cer-P, HexCer, GA1, GA2, GD1, GD2, GM1, GM2, GM3, GT1, or CE.

In some embodiments, the lipid set used in the predictive model is limited to those that have a higher probability to be found in human lipid pools. In other embodiments, the lipid set excludes very short and very long acyl chains (e.g., less that 10 carbons and more than 26 carbons within a chain). In still other embodiments, the lipid set lipids (e.g., GPLs or LGPL) are limited to those containing an even number of carbons in the acyl chain. In other embodiments, the lipid set included one or more lipids listed in Table 2.

**Table 2**

| Lipid Class | Lipid Group | Abbrev | Acyl Chain Range | Unsaturated sites (# double bonds) | Additional features or comments | Total # of structures considered |
|---|---|---|---|---|---|---|
| Fatty Acids | Fatty Acids | FA | 10-26 | 0-6 | | 79 |
| Glycerolipids | Monoacylglycerols | MAG | 10-26 | 0-6 | Even # of carbons only | 43 |
| Glycerolipids | Diacylglycerols | DAG | 22-46 | 0-12 | Even # of carbons only | 120 |
| Glycerolipids | Triacylglycerols | TAG | 44-66 | 0-18 | | 316 |
| Glycerophospholipids | Phosphatidylcholines | PC | 28-44 | 0-12 | Even # of carbons only | 77 |
| Glycerophospholipids | Phosphatidylethanolamines | PE | 28-44 | 0-12 | Even # of carbons only | 77 |
| Glycerophospholipids | Phosphatidylserines | PS | 28-44 | 0-12 | Even # of carbons only | 77 |
| Glycerophospholipids | Phosphatidylinositols | PI | 28-44 | 0-12 | Even # of carbons only | 77 |
| Glycerophospholipids | Phosphatidylglycerols | PG | 28-44 | 0-12 | Even # of carbons only | 77 |
| Glycerop hospholipids | Phosphatidic acid | PA | 28-44 | 0-12 | Even # of carbons only | 77 |
| Lysoglycerophospholipids | Lysophosphatidylcholines | LysoPC | 10-26 | 0-6 | Even # of carbons only | 43 |
| Lysoglycerophospholipids | Lysophosphatidylethanolamines | LysoPE | 10-26 | 0-6 | Even # of carbons only | 43 |
| Lysoglycerophospholipids | Lysophosphatidylserines | LysoPS | 10-26 | 0-6 | Even # of carbons only | 43 |
| Lysoglycerophospholipids | Lysophosphatidylinositols | LysoPI | 10-26 | 0-6 | Even # of carbons only | 43 |
| Lysoglycerophospholipids | Lysophosphatidylglycerols | LysoPG | 10-26 | 0-6 | Even # of carbons only | 43 |
| Lysoglycerophospholipids | Lysophosphatidic acid | LysoPA | 10-26 | 0-6 | Even # of carbons only | 43 |
| Lysoglycerophospholipids | Lysophosphatidylcholines-pmg | LysoPC | 10-26 | 0-6 | Even # of carbons only | 43 |
| Lysoglycerophospholipids | Lysophosphatidylethanolamines-pmg | LysoPE | 10-26 | 0-6 | Even # of carbons only | 43 |
| Bis(monoacylglycero)phosphates | | BMP | 28-48 | 0-12 | | 211 |
| Sphingolipids | Sphingomyelins | SM | 30-42 | 0-3 | | 25 |
| Sphingolipids | Ceramides | Cer | 30-44 | 0-3 | | 33 |
| Sphingolipids | Ceramide phosphates | Cer-P | 30-44 | 0-3 | | 33 |
| Sphingolipids | Hexose Ceramides | HexCer | 30-44 | 0-3 | | 33 |
| Sphingolipids | Gangliosides A1 | GA1 | 28-46 | 0-2 | | 57 |
| Sphingolipids | Gangliosides A2 | GA2 | 28-46 | 0-2 | | 57 |
| Sphingolipids | Gangliosides D1 | GD1 | 28-46 | 0-2 | | 57 |
| Sphingolipids | Gangliosides D2 | GD2 | 28-46 | 0-2 | | 57 |
| Sphingolipids | Gangliosides M1 | GM1 | 28-46 | 0-2 | | 57 |
| Sphingolipids | Gangliosides M2 | GM2 | 28-46 | 0-2 | | 57 |
| Sphingolipids | Gangliosides M3 | GM3 | 28-46 | 0-2 | | 57 |
| Sphingolipids | Gangliosides A1 | GT1 | 28-46 | 0-2 | | 57 |
| Cholesterol Esters | Cholesterol Esters | CE | 10-26 | 0-6 | | 79 |

In some embodiments, the lipids in the lipid set can include one or more of BMP (30:1), BMP (32:1), BMP (34:1), BMP (35:4), BMP (36:3), BMP (37:1), BMP (37:7), BMP (38:1), BMP (38:2), BMP (38:4), BMP (39:1), BMP (39:4), BMP (40:1), BMP (40:2), BMP (40:3), BMP (40:4), BMP (40:7), BMP (42:10), BMP (42:2), BMP (42:5), BMP (44:8), CE (16:2), CE (18:2), CE (18:3), CE (18:4), CE (20:2), CE (20:4), CE (20:5), Cer (32:1), Cer (34:1), Cer (36:1), Cer (38:1), Cer (38:4), Cer (40:2), Cer (40:4), DAG (28:0), DAG (32:0), DAG (32:2), DAG (34:0), DAG (34:3), DAG (34:5), DAG (36:0), DAG (36:1), DAG (36:2), DAG (36:3), DAG (36:8), DAG (38:1), DAG (38:10), DAG (38:2), DAG (38:3), DAG (38:5), DAG (40:1), DAG (40:2), DAG (40:5), DH-LTB4 (20:3), FA (16:3), FA (19:1), GA2 (30:0), GA2 (33:2), GA2 (35:2), GA2 (37:2), GM3 (41:1), HexCer (32:1), HexDHCer (34:0), LacCer (30:0), LacCer (30:1), LacCer (32:2), LysoPA (16:2), LysoPA (16:3), LysoPA (18:1), LysoPA (22:0), LysoPA (22:1), LysoPC (16:0), LysoPC (18:0), LysoPC (18:1), LysoPC (18:4), LysoPC (20:4), LysoPC (20:5), LysoPC (26:6), LysoPC-pmg (12:0), LysoPC-pmg (18:3), LysoPC-pmg (24:4), LysoPC-pmg (26:0), LysoPE (10:1), LysoPE (16:2), LysoPE (18:2), LysoPE-pmg (18:4), LysoPS (24:1), MAG (18:0), MAG (20:3), MAG (24:2), PC (32:0), PC (32:1), PC (34:1), PC (34:1), PC (34:2), PC (34:3), PC (34:4), PC (34:6), PC (36:1), PC (36:2), PC (36:3), PC (36:4), PC (36:5), PC (36:6), PC (36:9), PC (38:2), PC (38:3), PC (38:4), PC (38:5), PC (38:6), PC (38:7), PC (38:8), PC (38:9), PC (40:5), PC (40:6), PC (40:7), PC (40:8), PC (40:9), PC (44:12), PC-pmg (30:1), PC-pmg (36:4), PC-pmg (38:5), PC-pmg (38:7), PC-pmg (40:11), PC-pmg (42:1), PE (34:7), PE (36:5), PE (36:7), PE (38:2), PE (38:3), PE (38:4), PE (38:5), PE (38:7), PE (40:4), PE (40:9), PE (42:12), PE (44:11), PE-pmg (28:2), PE-pmg (30:3), PE-pmg (34:6), PE-pmg (34:8), PE-pmg (36:5), PE-pmg (36:6), PE-pmg (40:7), PE-pmg (40:8), PE-pmg (42:10), PE-pmg (42:12), PE-pmg (42:4), PE-pmg (42:7), PE-pmg (42:8), PE-pmg (42:9), PE-pmg (44:10), PE-pmg (44:11), PE-pmg (44:12), PE-pmg (44:7), PE-pmg (44:8), PE-pmg (44:9), PGA1 (20:1), PGB1 (20:1), SM (34:1), SM (34:2), SM (36:1), SM (38:1), SM (40:1), SM (40:2), SM (42:1), SM (42:2), SM (42:3), Sphingosine (18:0), TAG (44:1), TAG (44:3), TAG (46:0), TAG (46:1), TAG (46:2), TAG (46:3), TAG (46:4), TAG (48:0), TAG (48:1), TAG (48:2), TAG (48:3), TAG (48:4), TAG (48:5), TAG (49:1), TAG (49:2), TAG (49:3), TAG (50:0), TAG (50:1), TAG (50:2), TAG (50:3), TAG (50:4), TAG (50:5), TAG (50:6), TAG (51:2), TAG (51:4), TAG (52:2), TAG (52:3), TAG (52:4), TAG (52:5), TAG (52:6), TAG (52:7), TAG (53:4), TAG (54:2), TAG (54:3), TAG (54:4), TAG (54:5), TAG (54:6), TAG (54:7), TAG (54:8), TAG (55:5), TAG (55:6), TAG (55:7), TAG (56:4), TAG (56:5), TAG (56:6), TAG (56:7), TAG (56:8), TAG (56:9), TAG (58:10), TAG (58:6), TAG (58:8), TAG (58:9), TAG (60:12), or TH-12-keto-LTB4(20:2).

In some embodiments (e.g., to determine lung cancer), the lipids in the lipid set include, but are not limited to LysoPA (22:0), PE-pmg (42:9), FA (16:3), FA (19:1), CE (18:2), PE-pmg (44:11), BMP (30:1), PE-pmg (44:12), BMP (42:10), BMP (36:3), PC (34:6), BMP (40:3), TAG (50:2), BMP (39:1), DAG (38:3), BMP (37:1), PC-pmg (40:11), DAG (40:5), DAG (32:2), TAG (46:2), BMP (42:5), PE-pmg (42:8), PC (44:12), GA2 (35:2), TAG (50:1), CE (20:5), DAG (40:2), TAG (49:2), LysoPE (18:2), BMP (40:7), DAG (36:8), LysoPC (18:1), PE-pmg (36:5), PE-pmg (42:7), DH-LTB4 (20:3), PGA1 (20:1), PGB1 (20:1), BMP (38:4), BMP (35:4), BMP (44:8), TAG (46:1), TAG (44:1), LysoPC (18:4), DAG (36:0), DAG (38:2), LysoPC (20:4), DAG (38:1), LysoPC (26:6), DAG (36:2), DAG (34:5), TAG (49:1), TAG (56:7), DAG (38:5), Cer (40:2), BMP (40:4), GA2 (30:0), LysoPC-pmg (12:0), LysoPC-pmg (26:0), PC-pmg (30:1), LysoPC (20:5), PE-pmg (44:10), PE-pmg (34:8), PE-pmg (44:7), GM3 (41:1), BMP (37:7), PC (38:9), CE (20:4), SM (36:1), LysoPC-pmg (18:3), TAG (54:2), PE (38:5), PC (34:4), PC (34:3), TAG (48:0), TAG (50:5), DAG (32:0), PC (36:3), LysoPA (18:1), TAG (48:3), TAG (50:4), TAG (54:3), LysoPA (16:3), PC (36:1), TAG (58:9), PE-pmg (36:6), TAG (54:7), TAG (56:5), SM (42:1), LysoPA (16:2), DAG (28:0), TAG (46:3), TAG (54:8), SM (42:2), PC (40:8), LysoPE (10:1), PE (44:11), TAG (56:9), PC (40:6), SM (40:1), PE (36:5), Cer (32:1), BMP (39:4), PE-pmg (34:6), DAG (34:3), TAG (54:4), TAG (54:6), TAG (52:6), PE (36:7), PC (38:4), DAG (36:3), PC (36:2), PC (38:6), Cer (40:4), TAG (52:4), MAG (24:2), TAG (54:5), PC (36:5), TAG (50:3), TAG (52:5), MAG (18:0), LysoPA (22:1), TAG (52:3), PC (36:4), PC (40:7), PC (34:2), PC (34:1), Cer (34:1), PC (38:7), Cer (36:1), Cer (38:4), PC (38:5), Cer (38:1), or TAG (44:3).

In some embodiments (e.g., to determine lung cancer), the lipids in the lipid set include, but are not limited to LysoPA (22:0), PE-pmg (42:9), FA (16:3), FA (19:1), CE (18:2), Cer (36:1), Cer (38:4), PC (38:5), Cer (38:1), or TAG (44:3).

In some embodiments (e.g., to determine lung cancer), the lipids in the lipid set include, but are not limited to LysoPA (22:0), PE-pmg (42:9), FA (16:3), FA (19:1), CE (18:2), PE-pmg (44:11), BMP (30:1), PE-pmg (44:12), BMP (42:10), BMP (36:3), PC (34:6), BMP (40:3), TAG (50:2), BMP (39:1), DAG (38:3), BMP (37:1), PC-pmg (40:11), DAG (40:5), DAG (32:2), TAG (46:2), BMP (42:5), PE-pmg (42:8), PC (44:12), GA2 (35:2), TAG (50:1), PE (36:7), PC (38:4), DAG (36:3), PC (36:2), PC (38:6), Cer (40:4), TAG (52:4), MAG (24:2), TAG (54:5), PC (36:5), TAG (50:3), TAG (52:5), MAG (18:0), LysoPA (22:1), TAG (52:3), PC (36:4), PC (40:7), PC (34:2), PC (34:1), Cer (34:1), PC (38:7), Cer (36:1), Cer (38:4), PC (38:5), Cer (38:1), or TAG (44:3).

In some embodiments (e.g., to determine lung cancer), the lipids in the lipid set include, but are not limited to TAG (44:3), PC (36:5), PC (38:5), Cer (38:4), PE-pmg (42:9), PC (38:7), LysoPA (22:0), Cer (38:1), Cer (34:1), Cer (36:1), PC (40:7), TAG (54:5), TAG (54:6), CE (18:2), PC (36:4), FA (16:3), PE-pmg (44:11), TAG (52:5), Cer (40:4), CE (20:5), PC (38:6), TAG (50:2), MAG (18:0), FA (19:1), TAG (52:2), LysoPA (22:1), MAG (24:2), TAG (54:7), TAG (50:3), TAG (50:1), DAG (36:3), PC (34:1), TAG (52:6), BMP (30:1), PE-pmg (44:12), CE (20:4), BMP (40:3), PE (44:11), PC (40:8), TAG (56:9), PE-pmg (34:6), PE (36:7), PE (36:5), TAG (56:7), TAG (56:8), DAG (34:3), TAG (56:6), BMP (42:10), TAG (52:3), BMP (39:4), BMP (36:3), TAG (54:3), TAG (56:5), TAG (54:8), PC (34:6), PC (40:6), DAG (36:0), LysoPE (10:1), DAG (40:5), Cer (32:1), TAG (50:5), TAG (50:4), PE-pmg (36:6), BMP (42:5), TAG (46:3), or PE (38:5).

In some embodiments (e.g., to determine lung cancer), the lipids in the lipid set include, but are not limited to TAG (44:3), PC (36:5), PC (38:5), Cer (38:4), PE-pmg (42:9), PC (38:7), LysoPA (22:0), Cer (38:1), Cer (34:1), or Cer (36:1).

In some embodiments (e.g., to determine breast cancer), the lipids in the lipid set include, but are not limited to LysoPA (22:1), PE-pmg (42:9), CE (20:5), TAG (52:3), LysoPA (22:0), TAG (52:2), DAG (32:0), TAG (54:4), DAG (34:0), DAG (36:0), TAG (54:3), PE-pmg (44:11), TAG (44:3), BMP (30:1), TAG (52:4), BMP (40:3), PC (36:5), PC (36:9), PC (34:6), PE-pmg (44:12), BMP (42:10), BMP (36:3), PC-pmg (40:11), FA (16:3), DAG (38:3), TAG (54:2), BMP (42:5), HexDHCer (34:0), DAG (40:5), Cer (40:4), PC-pmg (30:1), PC (44:12), PE (38:5), PE (42:12), TAG (48:0), PE (36:5), PE-pmg (34:6), MAG (24:2), PE-pmg (36:6), TAG (56:4), TAG (49:3), CE (18:3), FA (19:1), DAG (38:5), TAG (50:0), BMP (38:4), TAG (46:3), TAG (51:4), BMP (35:4), PE-pmg (36:5), PE (44:11), DAG (38:2), TAG (48:3), CE (20:4), CE (18:4), LysoPC-pmg (18:3), BMP (44:8), LysoPC (20:4), TAG (60:12), LysoPC (18:4), PE-pmg (28:2), PE (40:4), PC-pmg (42:1), Sphingosine (18:0), LacCer (32:2), LysoPC (18:0), PE (38:3), MAG (20:3), SM (34:2), PC (40:5), SM (42:1), PE (38:7), LacCer (30:1), TAG (44:1), TAG (58:8), PC (40:9), CE (16:2), TAG (58:10), PE-pmg (44:10), SM (40:2), TAG (50:4), LysoPE-pmg (18:4), GA2 (37:2), PC-pmg (38:5), PC-pmg (36:4), PC (38:2), LacCer (30:0), GA2 (33:2), SM (42:3), PE (38:4), TAG (46:1), PC (32:1), BMP (42:2), LysoPC (16:0), SM (38:1), PE (38:2), TAG (50:3), TAG (58:9), PC (40:6), TAG (48:1), TAG (50:2), BMP (38:2), PE-pmg (40:7), PE-pmg (42:10), LysoPC-pmg (24:4), PC (34:3), PE-pmg (44:9), SM (36:1), PE-pmg (42:12), TAG (48:2), BMP (40:1), PE-pmg (44:8), DAG (36:1), TAG (56:7), LysoPC (26:6), PE-pmg (40:8), CE (18:2), PC (32:0), TAG (54:8), Cer (36:1), GA2 (35:2), TAG (56:6), TAG (56:9), DAG (36:8), PE-pmg (42:7), BMP (40:2), PC (38:3), PC (40:7), DAG (32:2), SM (42:2), SM (40:1), MAG (18:0), TAG (56:8), PE-pmg (42:8), TAG (52:5), DAG (40:1), PC (36:1), SM (34:1), DAG (38:1), TAG (54:7), Cer (38:1), BMP (39:1), BMP (37:1), Cer (34:1), TAG (54:6), PC (38:4), TAG (54:5), PC (36:3), PC (36:4), PC (36:2), PC (34:2), or PC (34:1).

In some embodiments (e.g., to determine breast cancer), the lipids in the lipid set include, but are not limited to LysoPA (22:1), PE-pmg (42:9), CE (20:5), TAG (52:3), LysoPA (22:0), PC (36:3), PC (36:4), PC (36:2), PC (34:2), or PC (34:1).

In some embodiments (e.g., to determine breast cancer), the lipids in the lipid set include, but are not limited to LysoPA (22:1), PE-pmg (42:9), CE (20:5), TAG (52:3), LysoPA (22:0), TAG (52:2), DAG (32:0), TAG (54:4), DAG (34:0), DAG (36:0), TAG (54:3), PE-pmg (44:11), TAG (44:3), BMP (30:1), TAG (52:4), BMP (40:3), PC (36:5), PC (36:9), PC (34:6), PE-pmg (44:12), BMP (42:10), BMP (36:3), PC-pmg (40:11), FA (16:3), DAG (38:3), PC (40:7), DAG (32:2), SM (42:2), SM (40:1), MAG (18:0), TAG (56:8), PE-pmg (42:8), TAG (52:5), DAG (40:1), PC (36:1), SM (34:1), DAG (38:1), TAG (54:7), Cer (38:1), BMP (39:1), BMP (37:1), Cer (34:1), TAG (54:6), PC (38:4), TAG (54:5), PC (36:3), PC (36:4), PC (36:2), PC (34:2), or PC (34:1).

In some embodiments (e.g., to determine breast cancer), the lipids in the lipid set include, but are not limited to PC (34:2), PC (34:1), PC (36:2), PC (36:4), PC (36:3), PC (38:4), LysoPA (22:1), PE-pmg (42:9), LysoPA (22:0), CE (20:5), Cer (36:1), CE (18:2), DAG (34:0), SM (34:1), DAG (32:0), PE-pmg (40:8), PC (38:3), DAG (36:0), PC (36:1), TAG (54:5), TAG (54:6), PE-pmg (44:11), PE-pmg (42:8), TAG (52:2), SM (42:2), PC (38:6), TAG (54:7), PC (40:6), PC (40:7), LysoPC (16:0), FA (16:3), TAG (52:5), TAG (44:3), BMP (38:2), BMP (30:1), SM (40:1), PE-pmg (42:10), BMP (40:2), PE-pmg (40:7), SM (36:1), PE (38:2), PC (34:3), PC (36:5), PC (32:0), PC (32:1), BMP (37:1), BMP (40:3), PC (36:9), SM (42:3), PC-pmg (36:4), PC-pmg (38:5), PC (40:9), TAG (54:3), PE-pmg (44:12), BMP (36:3), FA (19:1), BMP (39:1), TAG (50:3), BMP (42:10), PC (34:6), GA2 (35:2), TAG (58:9), PE-pmg (42:7), or LysoPC (18:0).

In some embodiments (e.g., to determine breast cancer), the lipids in the lipid set include, but are not limited to PC (34:2), PC (34:1), PC (36:2), PC (36:4), PC (36:3), PC (38:4), LysoPA (22:1), PE-pmg (42:9), LysoPA (22:0), or CE (20:5).

In some embodiments (e.g., to determine breast cancer and lung cancer), the lipids in the lipid set include, but are not limited to LysoPA (22:1), PC (36:5), TAG (52:3), PC (38:5), CE (20:5), TAG (44:3), PC (38:7), TAG (52:2), TAG (54:4), TAG (52:4), Cer (38:4), DAG (32:0), MAG (24:2), DAG (34:0), TAG (54:3), PC (38:6), Cer (40:4), TAG (52:6), PE-pmg (34:6), PE (36:5), DAG (36:0), Cer (36:1), CE (20:4), PC (36:9), PE-pmg (36:6), PE (38:5), PE (36:7), TAG (50:5), TAG (46:3), TAG (48:3), DAG (36:3), TAG (48:0), PC (40:7), CE (18:3), LysoPE (10:1), TAG (56:5), TAG (52:7), PE (44:11), Cer (38:1), TAG (54:2), LysoPA (16:2), TAG (52:5), TAG (48:4), LysoPA (16:3), DAG (28:0), LysoPC-pmg (18:3), HexDHCer (34:0), Cer (32:1), DAG (34:3), TAG (50:3), BMP (39:4), LysoPA (18:1), TAG (49:3), DAG (38:3), MAG (18:0), TAG (56:4), PC (40:8), PE-pmg (42:4), TAG (50:0), TAG (48:5), TAG (50:6), DAG (38:10), BMP (34:1), PC (36:6), BMP (37:7), TAG (55:6), PC (34:4), BMP (32:1), PC (38:8), PC-pmg (38:7), TAG (46:0), TAG (46:4), PC (38:9), TAG (53:4), TAG (55:5), TAG (55:7), TAG (58:6), TAG (58:9), BMP (38:1), TH-12-keto-LTB4(20:2), PC (34:6), HexCer (32:1), LysoPE (16:2), PE (34:7), LysoPS (24:1), PC (40:5), LysoPC (18:0), TAG (51:2), PE (38:3), Sphingosine (18:0), PC-pmg (38:5), PC-pmg (36:4), BMP (40:4), LacCer (30:1), SM (40:2), BMP (30:1), PC-pmg (42:1), PE-pmg (28:2), PE-pmg (30:3), PE (38:2), CE (20:2), DAG (34:5), BMP (42:2), Cer (34:1), PC (32:1), PE-pmg (44:12), GA2 (37:2), GA2 (33:2), LysoPA (22:0), DAG (40:2), TAG (56:7), TAG (54:5), LysoPE (18:2), LysoPE-pmg (18:4), CE (16:2), TAG (56:6), BMP (40:7), PE-pmg (40:7), BMP (38:2), MAG (20:3), TAG (49:2), PE (38:4), TAG (49:1), PE-pmg (42:10), DAG (36:2), BMP (42:10), TAG (44:1), LysoPC (16:0), PC (38:2), SM (42:2), PE-pmg (44:9), BMP (40:1), PE-pmg (44:8), PE-pmg (44:11), TAG (46:2), LysoPC-pmg (24:4), SM (40:1), PE-pmg (42:9), DAG (40:5), PE (40:9), PE-pmg (40:8), PE-pmg (42:12), PC (38:3), TAG (46:1), BMP (40:2), PC (32:0), TAG (56:8), PE-pmg (42:7), DAG (36:1), GA2 (35:2), LysoPC (26:6), TAG (54:6), TAG (48:1), TAG (54:7), PE-pmg (42:8), DAG (36:8), PC (36:1), SM (34:1), TAG (48:2), DAG (40:1), DAG (32:2), TAG (50:1), FA (16:3), PC (36:4), DAG (38:1), PC (38:4), FA (19:1), PC (36:3), PC (36:2), BMP (37:1), TAG (50:2), BMP (39:1), PC (34:2), CE (18:2), or PC (34:1).

In some embodiments (e.g., to determine breast cancer and lung cancer), the lipids in the lipid set include, but are not limited to LysoPA (22:1), PC (36:5), TAG (52:3), PC (38:5), CE (20:5), TAG (50:2), BMP (39:1), PC (34:2), CE (18:2), or PC (34:1).

In some embodiments (e.g., to determine breast cancer and lung cancer), the lipids in the lipid set include, but are not limited to LysoPA (22:1), PC (36:5), TAG (52:3), PC (38:5), CE (20:5), TAG (44:3), PC (38:7), TAG (52:2), TAG (54:4), TAG (52:4), Cer (38:4), DAG (32:0), MAG (24:2), DAG (34:0), TAG (54:3), PC (38:6), Cer (40:4), TAG (52:6), PE-pmg (34:6), PE (36:5), DAG (36:0), Cer (36:1), CE (20:4), PC (36:9), PE-pmg (36:6), LysoPC (26:6), TAG (54:6), TAG (48:1), TAG (54:7), PE-pmg (42:8), DAG (36:8), PC (36:1), SM (34:1), TAG (48:2), DAG (40:1), DAG (32:2), TAG (50:1), FA (16:3), PC (36:4), DAG (38:1), PC (38:4), FA (19:1), PC (36:3), PC (36:2), BMP (37:1), TAG (50:2), BMP (39:1), PC (34:2), CE (18:2), or PC (34:1).

In some embodiments (e.g., to determine breast cancer and lung cancer), the lipids in the lipid set include, but are not limited to PC (34:2), PC (36:2), TAG (44:3), CE (18:2), PC (34:1), LysoPA (22:1), PC (36:5), Cer (36:1), CE (20:5), PC (36:3), PC (38:4), PC (36:4), Cer (38:4), PC (38:5), PC (38:7), Cer (38:1), TAG (50:2), Cer (34:1), SM (34:1), Cer (40:4), MAG (18:0), MAG (24:2), PC (38:3), PE-pmg (40:8), PE-pmg (42:8), TAG (50:1), DAG (32:0), PC (36:1), DAG (34:0), LysoPC (16:0), PE-pmg (34:6), DAG (36:3), PC (36:9), PE (36:5), TAG (52:6), FA (19:1), PE-pmg (44:11), BMP (38:2), PE (44:11), TAG (48:2), SM (42:2), BMP (40:2), PE-pmg (42:10), PE (36:7), PE-pmg (40:7), BMP (39:1), BMP (37:1), PE-pmg (36:6), PE (38:5), PC (32:0), PE (38:2), GA2 (35:2), DAG (34:3), PE-pmg (44:12), MAG (16:0), PC (32:1), LysoPE (10:1), SM (36:1), BMP (39:4), TAG (56:7), or PE-pmg (42:9).

In some embodiments (e.g., to determine breast cancer and lung cancer), the lipids in the lipid set include, but are not limited to PC (34:2), PC (36:2), TAG (44:3), CE (18:2), PC (34:1), LysoPA (22:1), PC (36:5), Cer (36:1), CE (20:5), or PC (36:3).

In some embodiments, the number of lipids in the lipid set can include at least 10, at least 50, at least 100, at least 150, at least 200, at least 500, or at least 1000 lipids. In some embodiments, the number of lipids in the lipid set can include no more than 200, no more than 500, no more than 1,000, no more than 5,000, no more than 10,000, or no more than 100,000 lipids.

Animals include but are not limited to primates (e.g., humans), canine, equine, bovine, porcine, ovine, avian, or mammalian. Animals include those as pets or in zoos and include domesticated swine and horses (including race horses). In addition, any animal connected to commercial activities are also included such as those animals connected to agriculture and aquaculture and other activities in which disease monitoring, diagnosis, and therapy selection are routine practice in husbandry for economic productivity and/or safety of the food chain. In some embodiments, the animal is a human, dog, cat, horse, cow, pig, sheep, chicken, turkey, mouse, or rat.

The cancer types (including cancerous tumors) can include but are not limited to carcinomas, sarcomas, hematologic cancers, neurological malignancies, thyroid cancer, neuroblastoma, melanoma, renal cell carcinoma, hepatocellular carcinoma, breast cancer, colon cancer, lung cancer, pancreatic cancer, brain cancer, prostate cancer, chronic lymphocytic leukemia, acute lymphoblastic leukemia, rhabdomyosarcoma, Glioblastoma multiforme, meningioma, bladder cancer, gastric cancer, Glioma, oral cancer, nasopharyngeal carcinoma, kidney cancer, rectal cancer, lymph node cancer, bone marrow cancer, stomach cancer, uterine cancer, leukemia, basal cell carcinoma, cancers related to epithelial cells, or cancers that can alter the regulation or activity of Pyruvate Carboxylase. Cancerous tumors include, for example, tumors associated with any of the above mentioned cancers.

In some embodiments, determining the presence or absence of one or more cancer types includes determining the presence or absence of each cancer type.

The amount of a lipid can be determined using any suitable technique, including, for example, any of the mass spectrometry methods described herein.

Using a mass spectrometry system, the mass spectrometry spectrum of the sample is obtained. The mass spectrometry system can comprise the usual components of a mass spectrometer (e.g., ionization source, ion detector, mass analyzer, vacuum chamber, and pumping system) and other components, including but not limited to separation systems, such as interfaced chromatography systems. The mass spectrometer can be any suitable mass spectrometer for determining a lipid amount. The mass analyzer system can include any suitable system including but not limited to, time of flight analyzer, quadrupole analyzer, magnetic sector, Orbitrap, linear ion trap, or fourier transform ion cyclotron resonance (FTICR). In some instances, the mass analyzer system (e.g., FTICR) has sufficient resolution to determine lipid identity without further experimental means. The ion source can include, but is not limited to electron impact ionization, electrospray, chemical ionization, photoionization, atmospheric pressure chemical ionization, collisional ionization, natural ionization, thermal ionization, fast atom bombardment ionization, particle-beam ionization, or matrix-assisted laser desorption ionization (MALDI). In some instances, electrospray can be a non-ionizing ion source. Interfaced chromatography systems can include any suitable chromatography system, including but not limited to gas chromatography (GC), liquid chromatography (LC), or ion mobility (which can be combined with LC or GC methods). In some instances, direct infusion can be used. In some instances the mass spectrometry system is GC/MS or LC/MS.

In some instances, once the MS spectra are obtained, the spectra can be analyzed to determine the identity and amount (e.g., the presence) of lipids in the sample.

For MS spectra, analysis can include any suitable analysis to determine the identity and/or amount of a lipid including analysis of one or more characteristics, which include but are not limited to comparison of masses to known masses, chromatographic retention times (e.g., for GC/MS or LC/MS), and mass fragmentation patterns. In some instances, the analysis can include a comparison of characteristics with that of a database (e.g., a database of standards). In some instances, PREMISE (Lane, A.N., T.W.-M. Fan, X. Xie, H.N. Moseley, and R.M. Higashi, Stable isotope analysis of lipid biosynthesis by high resolution mass spectrometry and NMR Anal. Chim. Acta, 2009. 651: p. 201-208.) can be used to identify lipids. The lipid amount (e.g., the relative amount) can be determined by any suitable method, including but not limited to the response function of the ion detector, by reference to spiked standards, or by isotope dilution.

In some embodiments, before the data are input into the predictive model, the data can be pre-processed. Pre-processing methods can include one or more of any suitable method such as normalization methods or scaling methods. In some embodiments, scaling methods can include but are not limited to centering, unit variance, unit variance without centering, Pareto, and Pareto without centering.

A predictive model can comprise any suitable model for determining the presence or absence of one or more cancer types. For example, the predictive model can comprise one or more dimension-reduction methods. In some embodiments, the predictive model comprises one or more of clustering methods (e.g, K-means clustering and K-nearest neighbor clustering), machine learning methods (e.g., artificial neural networks (ANN) and support vector machines (SVM)), principal component analysis (PCA), soft independent modeling of class analogy (SIMCA), partial least squares (PLS) regression, orthogonal least squares (OPLS) regression, partial least squares discriminant analysis (PLS-DA), or orthogonal partial least squares discriminant analysis (OPLS-DA). These techniques may, if desired, include or be supplemented with one or more suitable methods including mean centering, median centering, Pareto scaling, unit variance scaling, orthogonal signal correction, integration, differentiation, cross validation, and receiver operating characteristic (ROC) curves. In some embodiments, the predictive model can be developed using a set of training data, where the training data is designed to produce a set of applicable parameters and coefficients. In some embodiments, the set of parameters and coefficients can be used to determine if an animal has cancer and in some instances the type of cancer. The training data can include negative control data (e.g., when no cancer is present in the animal) and positive control data (where one or more cancer types are present in the animal). The training data set can be used to establish a predictive model that can determine two or more cancer types.

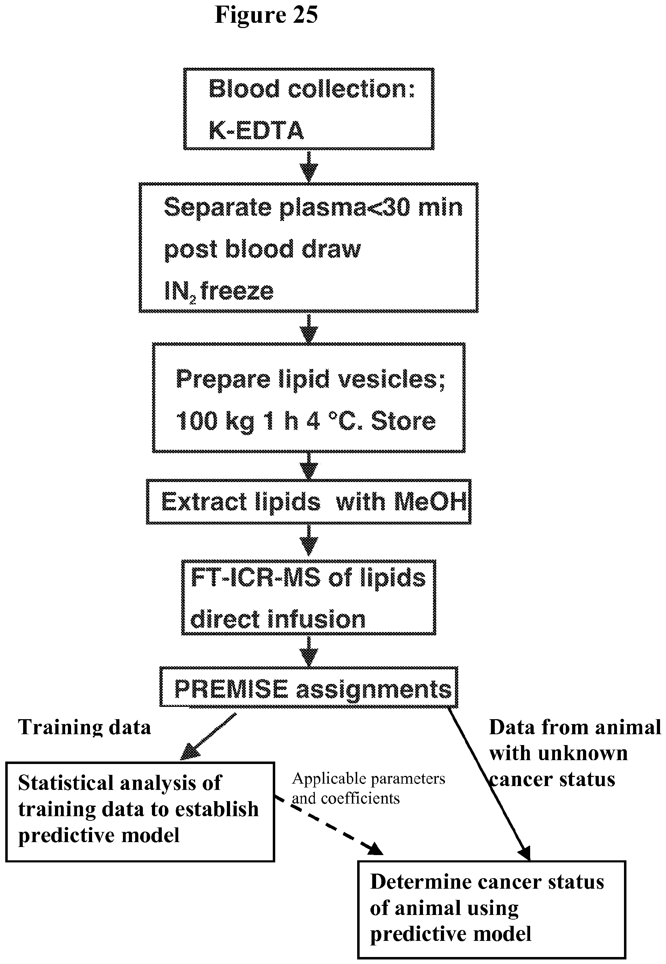

Figure 25 shows some embodiments of the method. It illustrates that, in some instances, once a predictive model has been established using a training data set, determination of the presence of one or more cancer types can be made from the predictive model without any additional training.

The methods of the present invention can further comprise the determination of the protein expression, gene expression, or both of proteins or their genes. Any suitable protein (or its gene) expression can be determined, including but not limited to pyruvate carboxylase, succinyl CoA synthetase, phosphoenolpyruvate carboxykinase, transketolase, transaldolase, pyruvate dehydrogenase, a dehydrogenase, glutaminase, isocitrate dehydrogenase, α-ketoglutarate dehydrogenase, mitochondrial malate dehydrogenase, succinate dehydrogenase, fumarate hydratase, hexokinase II, glyceraldehyde-3-phosphate dehydrogenase, phosphoglycerate kinase 1, lactate dehydrogenase 5, phosphofructokinase 1 and 2, glutathione peroxidase, or glutathione-S-transferase, or proteins associated with metabolic pathways such as, but are not limited to Krebs cycle (also known as the citric acid cycle), glycolysis, pentose phosphate pathway (oxidative and non-oxidative), gluconeogenesis, lipid biosynthesis, amino acid syntheses (e.g., synthesis of non-essential amino acids), catabolic pathways, urea cycle, Cori cycle or glutamate/glutamine cycle. Protein expression can be determined by any suitable technique including, but not limited to techniques comprising gel electrophoresis techniques (e.g., Western blotting), chromatographic techniques, antibody-based techniques, centrifugation techniques, or combinations thereof. Gene expression can be determined by any suitable technique including, but not limited to techniques comprising PCR based techniques (e.g., real-time PCR), gel electrophoresis techniques, chromatographic techniques, antibody-based techniques, centrifugation techniques, or combinations thereof. Methods for measuring gene expression can comprise measuring amounts of cDNA made from tissue-isolated RNA.

### EXAMPLES

**Plasma preparation:** Blood from humans diagnosed with breast cancer, diagnosed with the lung cancer non-small-cell lung carcinoma (NSCLC), and healthy (cancer-free) humans (also referred to as control or normal) was collected into K-EDTA containing vacutainer tubes (purple top) for anti-coagulation and immediately placed on ice and centrifuged at 3500xg for 15 min at 4°C. The supernatant (plasma) was aliquoted before flash freezing in liquid N₂ and kept at -80°C until fraction preparation.

**Fraction preparation:** Plasma was thawed and 0.8 ml transferred to a 1 ml polyallomer ultracentrifuge tubes using cold PBS to adjust for exact volume (entire volume of ultracentrifuge tubes must be filled to within 5 mm of the top to avoid collapse upon centrifugation). The rotor SWTi55 with buckets was precooled to 4°C. Ultracentrifuge tubes plus cap in bucket was weighed for adjusting all sample masses to within 10 mg of variation using PBS. Samples were then centrifuged in SWTi55 rotor at 70,000xg (27,172 rpm) for 1 h at 4°C. The supernatant was centrifuged again in SWTi55 at 100,000xg (32,477 rpm) for 1 h at 4°C to pellet the lipid microvesicle fraction. The pellet from the first centrifugation (microvesicle fraction) and the lipid microvesicle fraction pellet were washed in cold PBS by resuspension and centrifuged in SWTi55 at 100,000xg (32,477 rpm) for 1 h at 4°C. The supernatant was removed and both tubes were inverted on paper towel to drain excess PBS. The pellets were resuspended in 2x100 µl 18 MOhm water for transfer to 2 ml microfuge tubes and lyophilized overnight. The lyophilized pellets were kept at -80°C until lipid extraction.

**Lipid Extraction:** The lipid microvesicle fraction pellet was extracted in 0.5 ml methanol (mass spectrometry-grade)+1mM butylated hydroxytoluene by homogenization with 3x3 mm glass beads in a mixer mill (e.g. MM200, Retsch) for 1 minute at 30 Hz. The homogenate was then shaken in a rocker for 30 min before centrifugation at 14,000 rpm for 30 min at 4°C in a microcentrifuge. The supernatant was transferred into a 1.5 ml screw-cap glass vial with Teflon-faced silicone septum and the extract weight is recorded. The lipid extract was stored at -80°C until FT-ICR-MS analysis.

**FT-ICR-MS analysis:** Samples were diluted 1:5 in methanol+1 mM BHT+ 1ng/nl reserpine before analysis on a hybrid linear ion trap-FT-ICR mass spectrometer (ThermoFisher LTQ FT, Thermo Electron, Bremen, Germany), as described previously (Lane, A.N., T.W.-M. Fan, X. Xie, H.N. Moseley, and R.M. Higashi, Stable isotope analysis of lipid biosynthesis by high resolution mass spectrometry and NMR Anal. Chim. Acta, 2009. 651: p. 201-208). The FT-ICR-MS was equipped with a TriVersa NanoMate ion source (Advion BioSciences, Ithaca, NY) with an "A" electrospray chip (nozzle inner diameter 5.5µm). The TriVersa NanoMate was operated by applying 2.0 kV with 0.1 psi head pressure in positive ion mode and 1.5 kV and 0.5 psi in the negative mode. MS runs were recorded over a mass range from 150 to 1600 Da. Initially, low resolution MS scans were acquired for 1 min to ensure the stability of ionization, after which high mass accuracy data were collected using the FT-ICR analyzer where MS scans were acquired for 8.5 min and at the target mass resolution of 200,000 at 400 m/z. The AGC (automatic gain control) maximum ion time was set to 500ms (but typically utilized <10 ms) and five "µscans" were acquired for each saved spectrum; thus the cycle time for each transformed and saved spectrum was about 10 s. The LTQ-FT was tuned and calibrated according to the manufacturer's default standard recommendations, which achieved better than 1ppm mass accuracy. FT-ICR mass spectra were exported as exact mass lists into a spreadsheet file using QualBrowser 2.0 (Thermo Electron), typically exporting all of the observed peaks. Lipid species were assigned based on their accurate mass, by first applying a small (typically <0.0005) linear correction based on the observed mass of the internal standard (reserpine), then using an in-house software tool PREMISE (PRecaculated Exact Mass Isotopologue Search Engine) (Lane, A.N., T.W.-M. Fan, X. Xie, H.N. Moseley, and R.M. Higashi, Stable isotope analysis of lipid biosynthesis by high resolution mass spectrometry and NMR Anal. Chim. Acta, 2009. 651: p. 201-208) which was manually validated. PREMISE is a routine that matches observed with theoretical m/z values, subject to a selectable window that was 0.0014 Da or smaller. For lipids, the exact masses of a large number (>3500) of possible GPLs and their ion forms (principally H+ and Na+ - positive mode and -H+ - negative mode) were pre-calculated into a spreadsheet lookup table. The overall method was sufficient to assign a GPL to a particular total acyl chain length, degree of saturation, and headgroup identity.

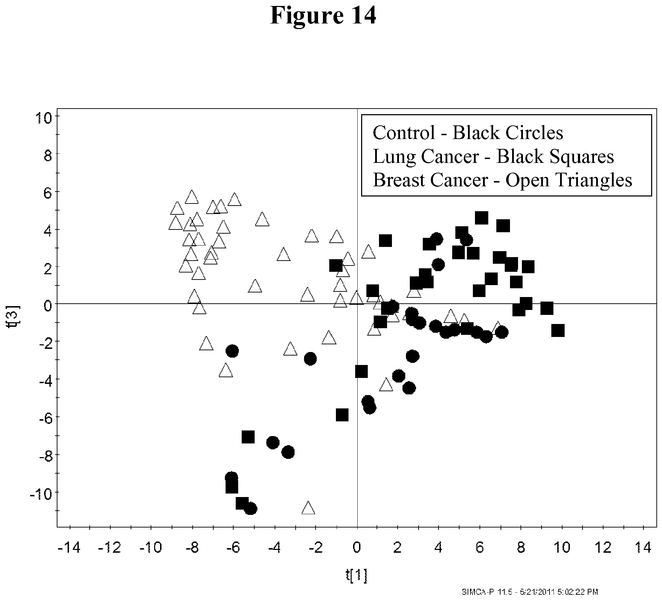

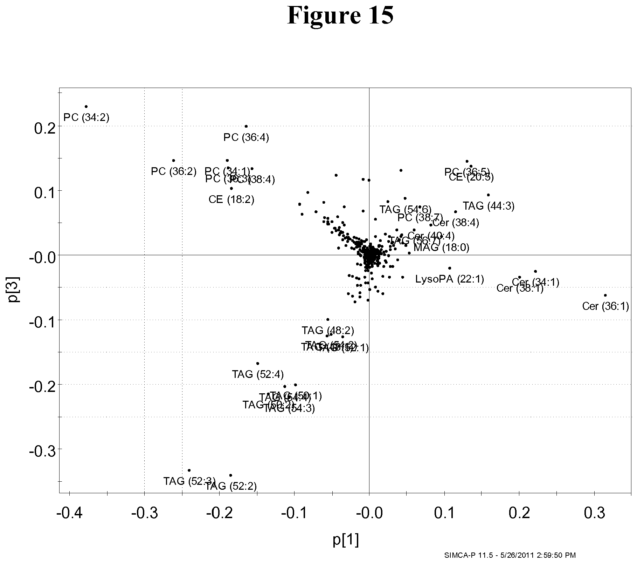

**Chemometrics Analysis:** The normalized FT-ICR-MS data were imported into SimcaP (version 11.5, Umetrics, Umeå, Sweden). The data were mean centered and scaled to Pareto variance (1/√sd). Principal component analysis (PCA) was then performed on the resulting data. This was done to find outlier samples or variables. No samples were determined to be outliers and one variable was. The variable Sphingosine (18:1) was excluded from further analysis. The PCA scores and loadings plots are shown in Figures 14 and 15.

Orthogonal partial least squares discriminant analysis (OPLS-DA) models were created from three subsets of data: normal (i.e., cancer-free) and lung cancer, normal (i.e., cancer-free) and breast cancer, and lung cancer and breast cancer. This analysis determined a dimension in multivariate space which maximizes group separation (for example, the control group and the lung cancer group) while removing one dimension orthogonal to the mentioned dimension which has maximum sample variance unrelated to class separation. This analysis determined which of the many variables are most different between the classes of data.

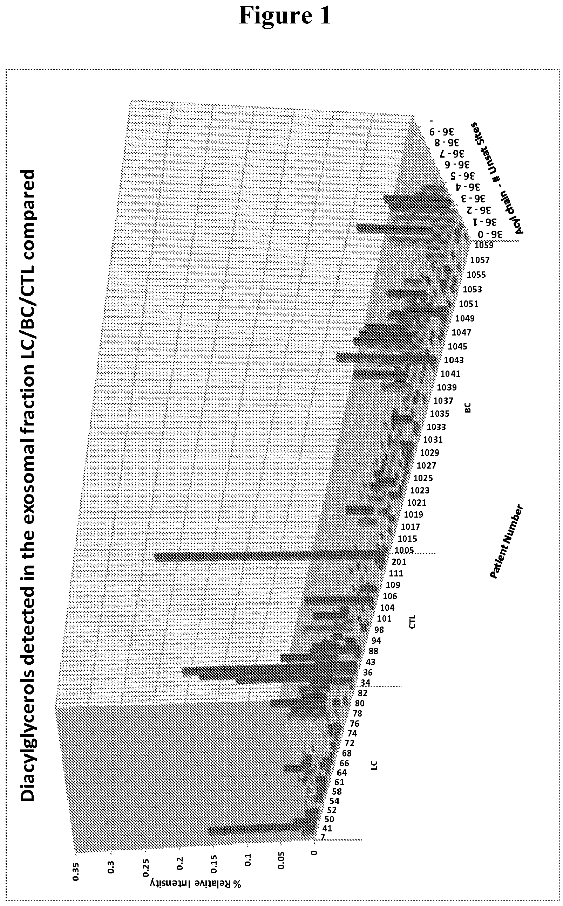

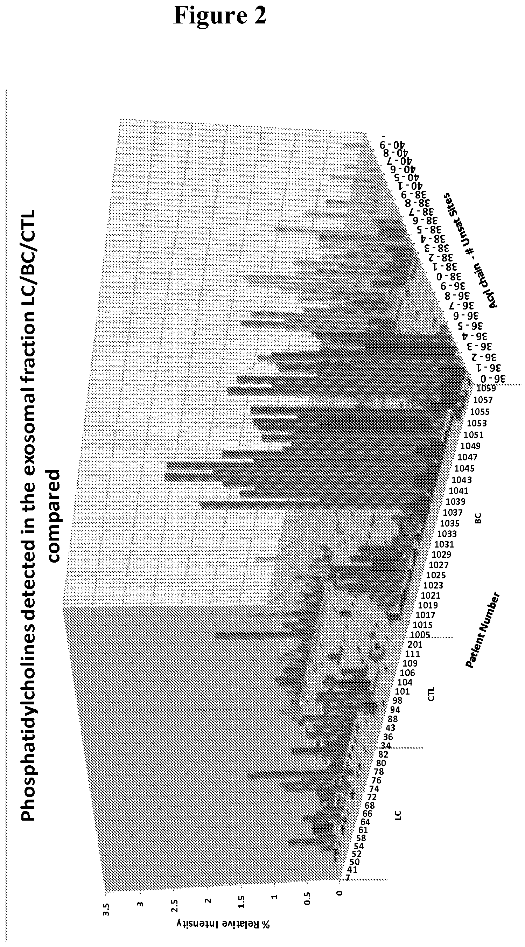

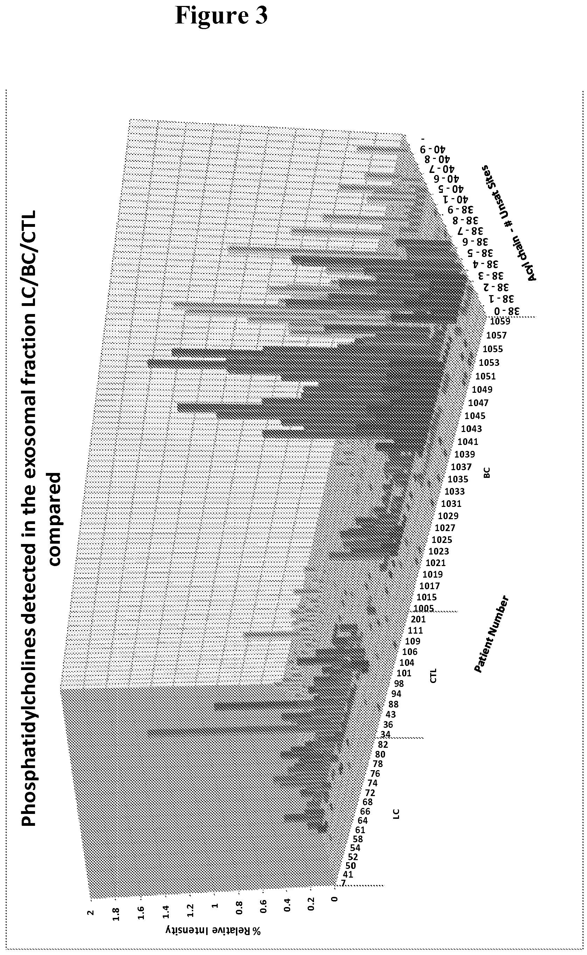

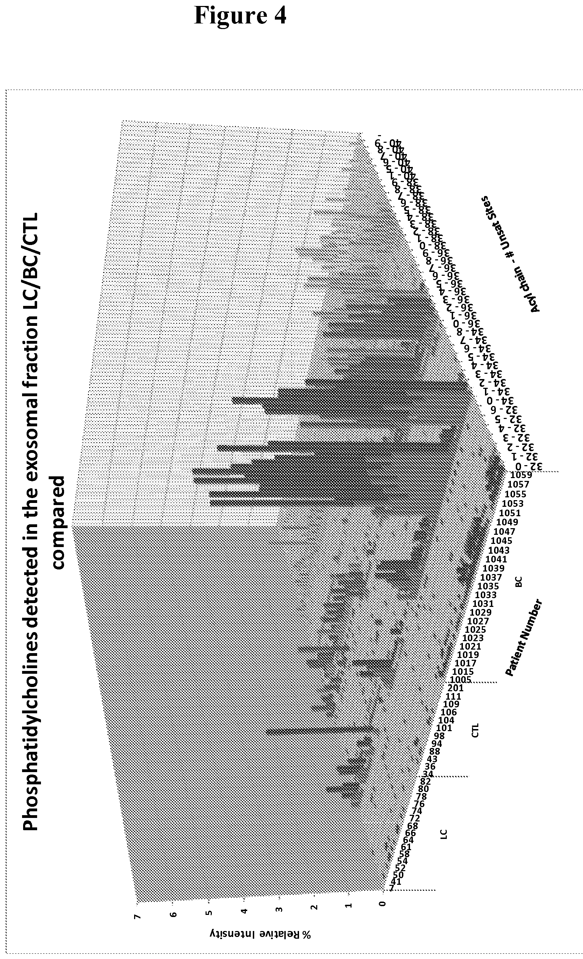

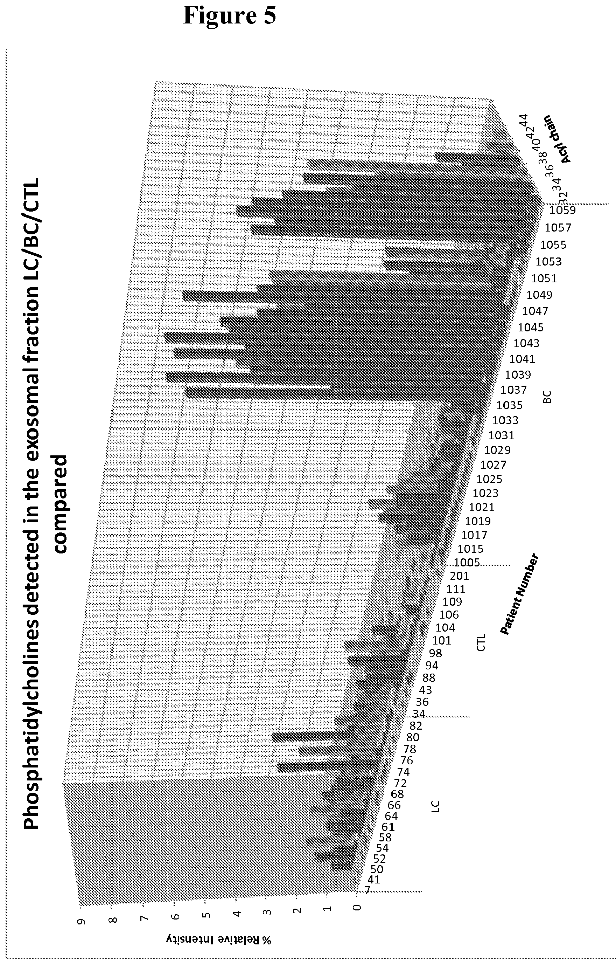

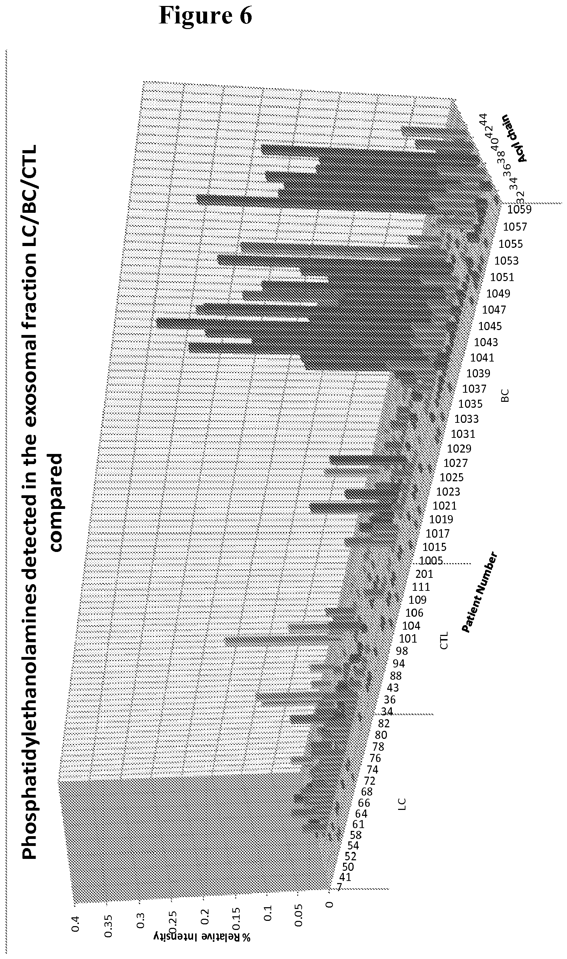

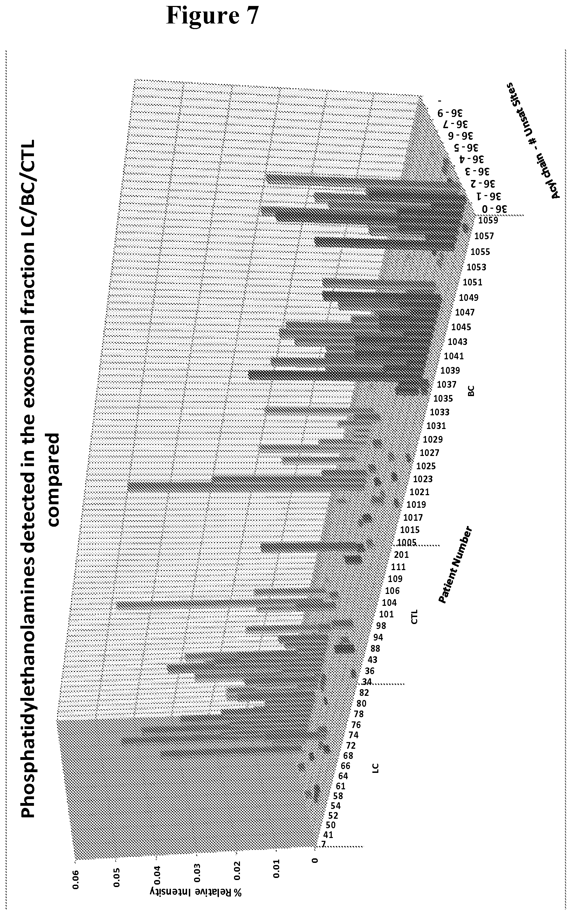

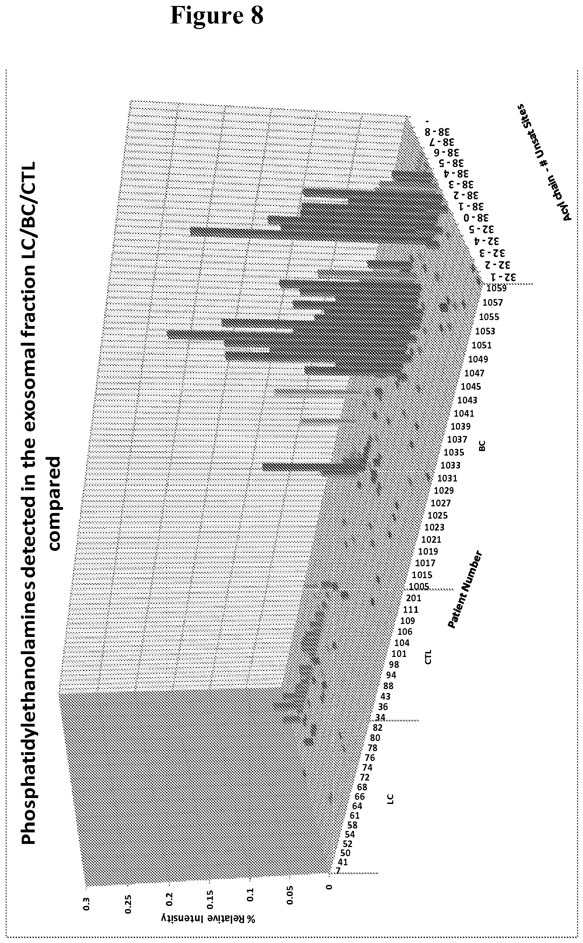

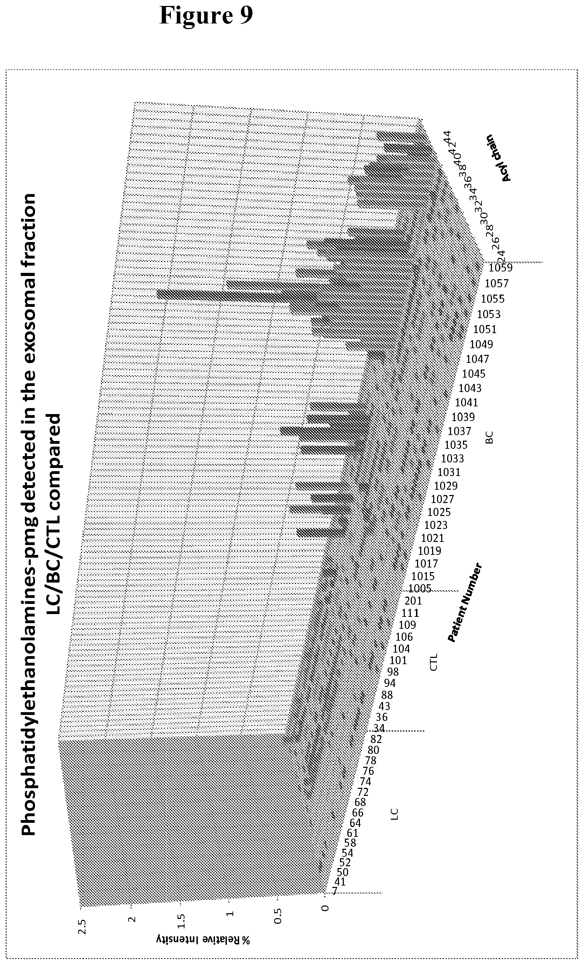

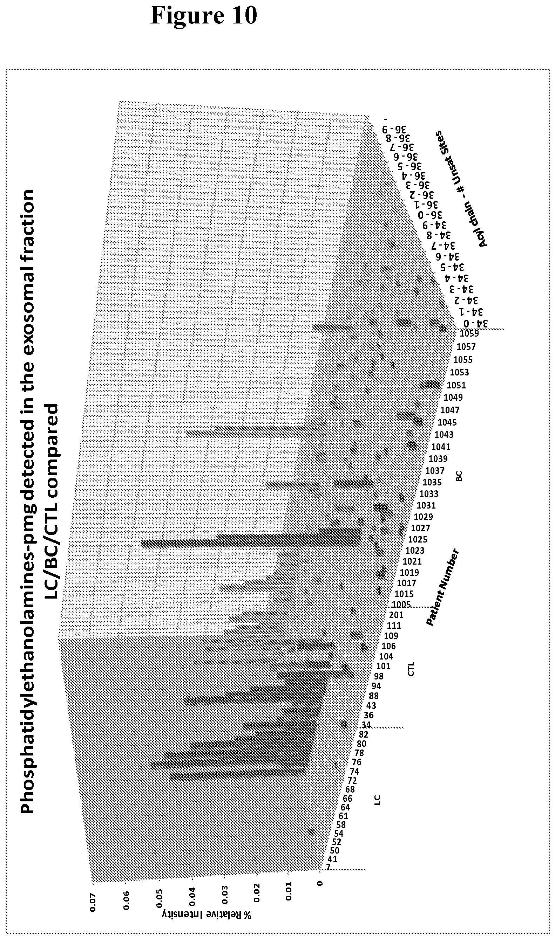

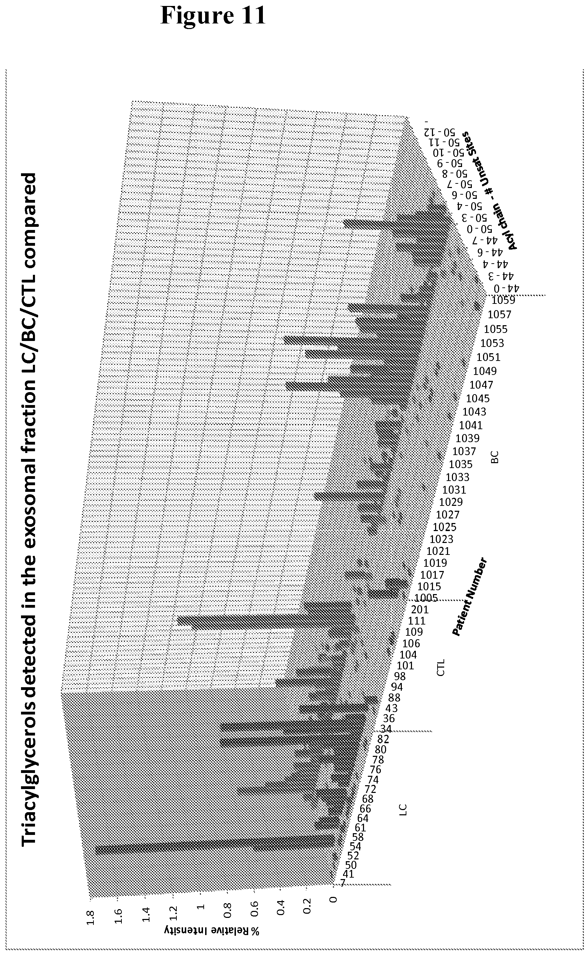

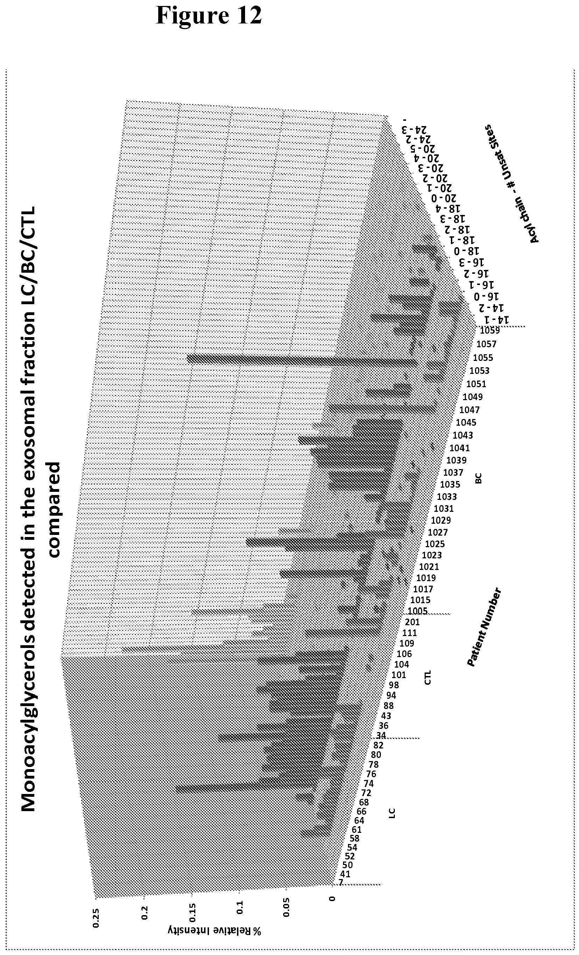

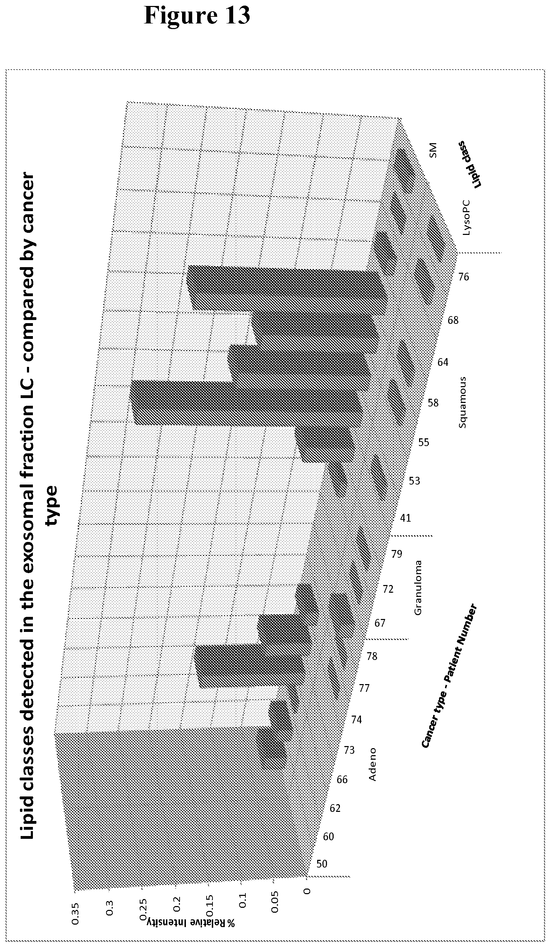

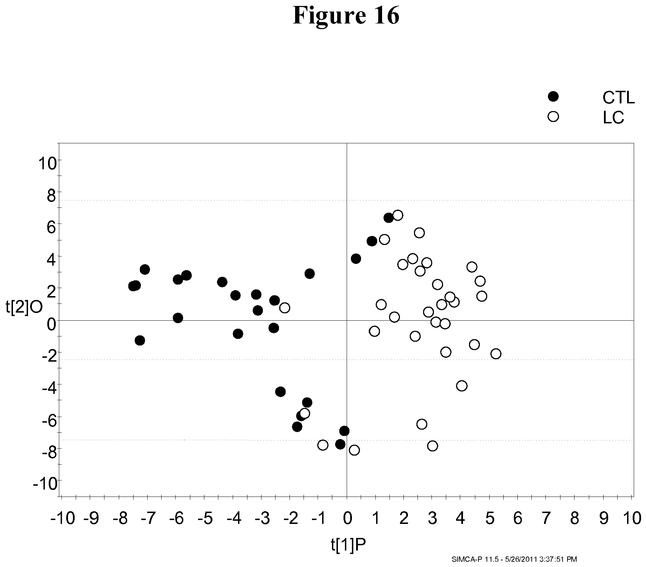

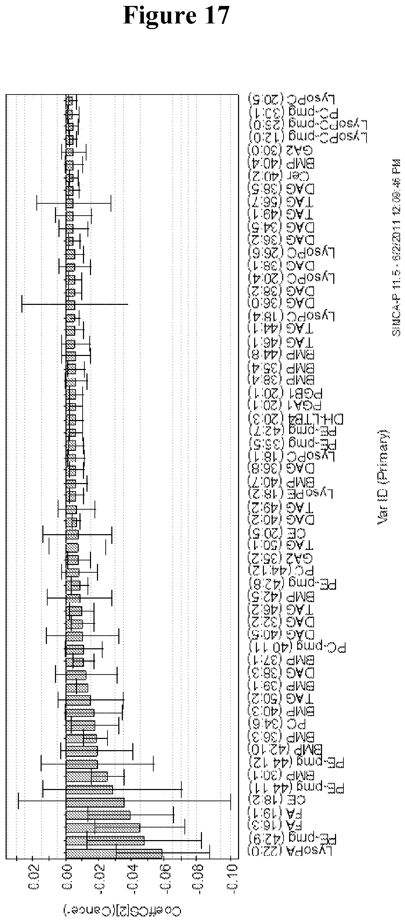

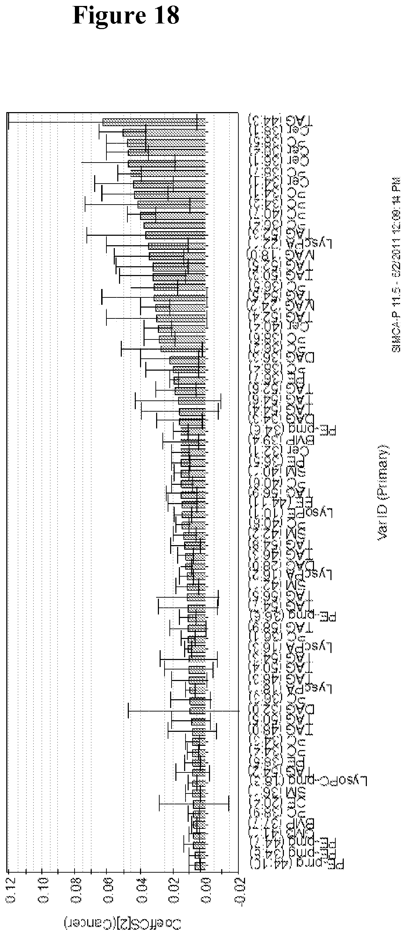

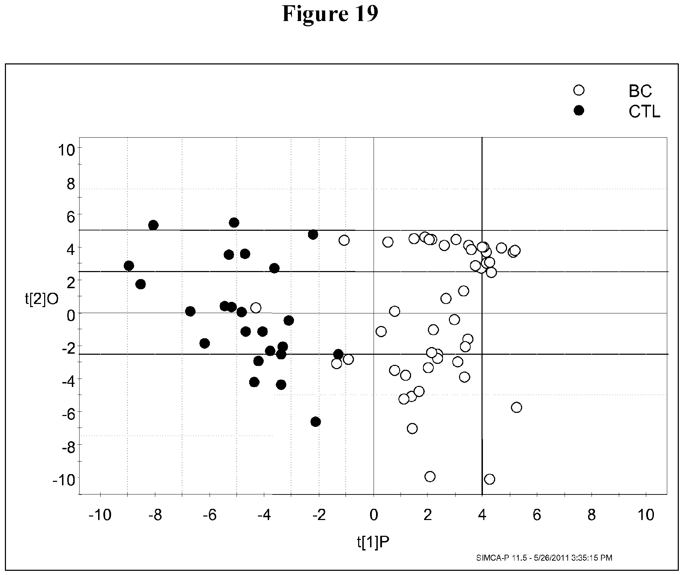

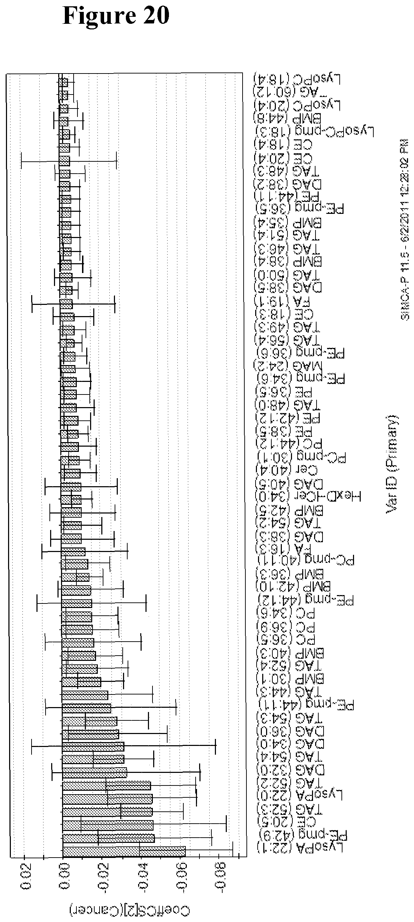

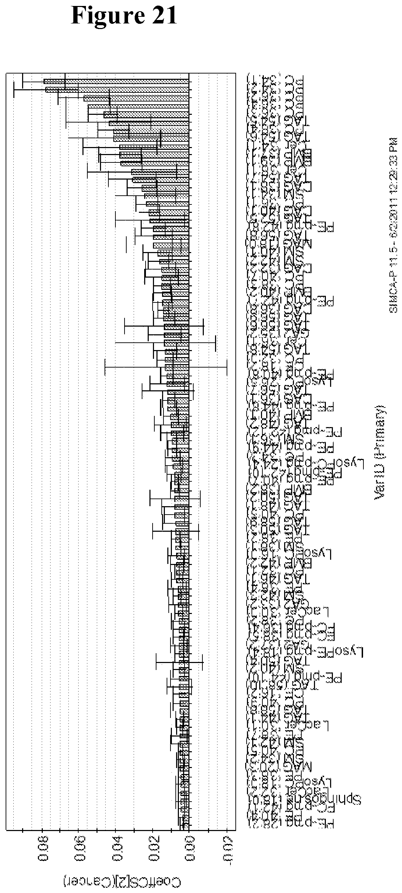

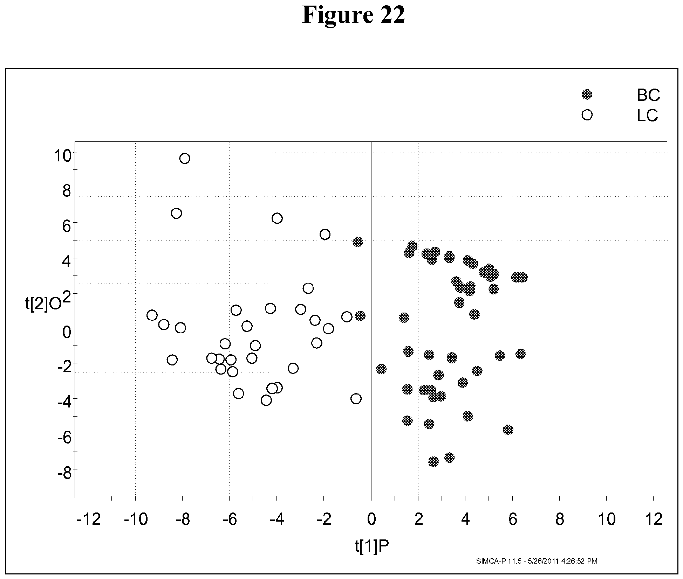

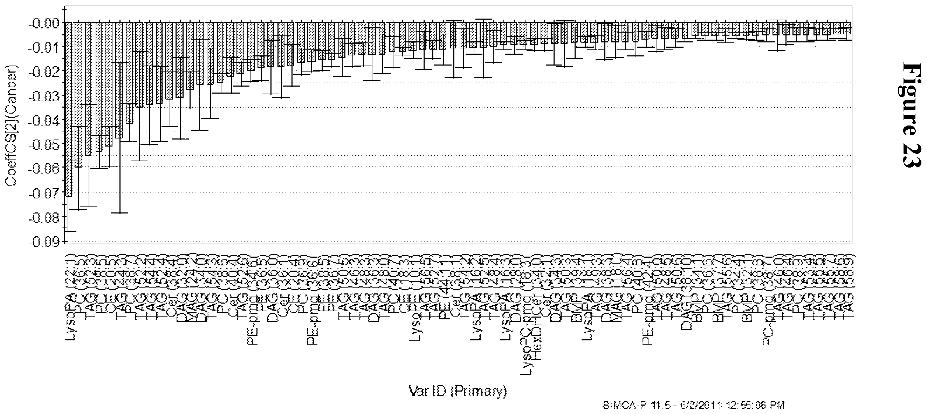

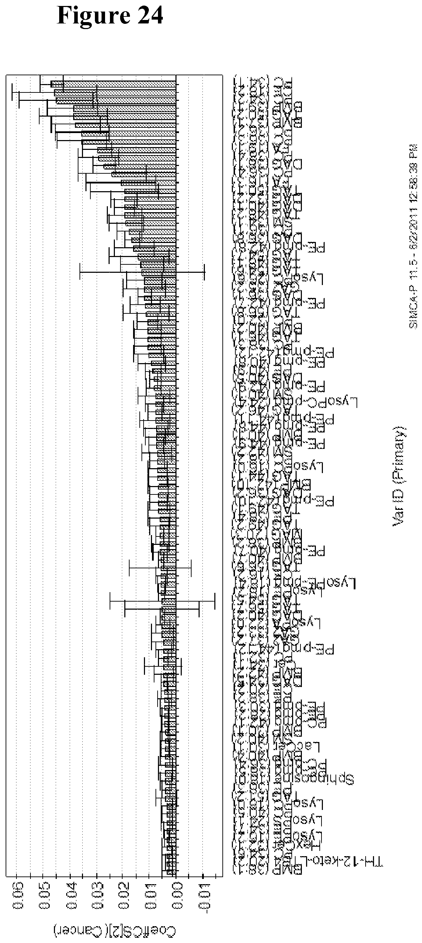

OPLS-DA showed good separation of the total lipid profiles from the three classes of plasma. Some of the lipids were essentially the same in each source of lipid microvesicle fraction (the common components). The major classes that gave rise to the discrimination were obtained from the loading and coefficient plots, which are visualized in the 3D plots shown in Figures 1-13. The OPLS-DA scores and coefficients plots are provided in Figures 16-24.

The intensities of the lipids species were normalized to the total lipid response to generate "mol fractions." Different lipid classes varied in their abundances, and within a class some acyl chain length and number of double bonds also vary substantially, which was part of the classification. Most of the variance arose from intersubject variability rather than analytical variance. The difference in abundance between classes for discrimination was >4 fold with a coefficient of variation within a class of up to 50%. For example, the PC (36:3) showed mean and sd of 1.38±0.34 (BrCA) versus 0.23±0.1 (healthy) versus 0.19±0.28 (NSCLC). This single instance provided statistical separation with p values of <0.0001 (BrCA versus healthy), <0.0001 (BrCa versus NSCLC). NSCLC versus healthy did not reach statistical significance. However, other lipids gave high statistical separation between NSCLC and healthy. Therefore, several classes together were used to discriminate among healthy individuals and those with cancer. Optimal segregation was achieved using sets of lipids where at least two of the subject classes differed with p values better than 0.01, and a minimum of ten such lipid classes were used for reliable discrimination

## Claims

1. A method for determining the presence or absence of at least one cancer type in an animal comprising
- determining lipids amounts of lipids in a lipid set in a sample from the animal, and
- determining the presence or absence of at least one cancer type in the animal with a predictive model;
wherein
- the lipid amounts of lipids in the lipid set comprise an input of the predictive model,
- the sample comprises a bodily fluid or treatment thereof;
- the at least one cancer type is selected from the group consisting of carcinomas, sarcomas, hematologic cancers, neurological malignancies, thyroid cancer, neuroblastoma, melanoma, renal cell carcinoma, hepatocellular carcinoma, breast cancer, colon cancer, lung cancer, pancreatic cancer, brain cancer, prostate cancer, chronic lymphocytic leukemia, acute lymphoblastic leukemia, rhabdomyosarcoma, Glioblastoma multiforme, meningioma, bladder cancer, gastric cancer, Glioma, oral cancer, nasopharyngeal carcinoma, kidney cancer, rectal cancer, lymph node cancer, bone marrow cancer, stomach cancer, uterine cancer, leukemia, basal cell carcinoma, cancers related to epithelial cells, cancers that can alter the regulation or activity of Pyruvate Carboxylase, and tumors associated with any of the aforementioned cancer types;
- the lipid set comprises a lipid from the class of BMP, preferably BMP (30:1), BMP (32:1), BMP (34:1), BMP (35:4), BMP (36:3), BMP (37:1), BMP (37:7), BMP (38:1), BMP (38:2), BMP (38:4), BMP (39:1), BMP (39:4), BMP (40:1), BMP (40:2), BMP (40:3), BMP (40:4), BMP (40:7), BMP (42:10), BMP (42:2), BMP (42:5), or BMP (44:8), and
- the predictive model comprises one or more of dimension reduction method, clustering method, machine learning method, principle component analysis, soft independent modeling of class analogy, partial least squares regression, orthogonal least squares regression, partial least squares discriminant analysis, orthogonal partial least squares discriminant analysis, mean centering, median centering, Pareto scaling, unit variance scaling, orthogonal signal correction, integration, differentiation, cross validation, or receiver operating characteristic curves.

2. The method of claim 1, wherein the bodily fluid is selected from the group consisting of plasma vomit, cerumen, gastric juice, breast milk, mucus, saliva, sebum, semen, sweat, tears, vaginal secretion, blood serum, aqueous humor, vitreous humor, endolymph, perilymph, peritoneal fluid, pleural fluid, cerebrospinal fluid, blood, plasma, nipple aspirate fluid, urine, stool, and bronchioalveolar lavage fluid.

3. The method of claim 1 or 2, wherein the sample comprises a lipid micro vesicle fraction.

4. The method of claim 1, 2 or 3, wherein the lipid set comprises at least 10, at least 50, at least 100, at least 200 or no more than 100,000 lipids.

5. The method of any preceding claim, wherein the lipid set further comprises one or more lipids selected from the one or more classes of lipids selected from the group consisting of CE, Cer, DAG, DH-LTB4, FA, GA2, GM3, HexCer, HexDHCer, LacCer, LysoPA, LysoPC, LysoPC-pmg, LysoPE, LysoPE-pmg, LysoPS, MAG, PC, PC-pmg, PE, PE-pmg, PGA1, PGB1, SM, Sphingosine, TAG, and TH-12-keto-LTB4.

6. The method of any of claims 1 to 4, wherein the lipid set further comprises one or more lipids selected from the one or more classes of lipids selected from the group consisting of FA, MAG, DAG, TAG, PI, PE, PS, PI, PG, PA, LysoPC, LysoPE, LysoPS, LysoPI, LysoPG, LysoPA, LysoPC, LysoPE, SM, Cer, Cer-P, HexCer, GA1, GA2, GD1, GD2, GM1, GM2, GM3, GT1, and CE.

7. The method of any of claims 1 to 4, wherein one or more lipids in the lipid set are further selected from the group consisting of CE (16:2), CE (18:2), CE (18:3), CE (18:4), CE (20:2), CE (20:4), CE (20:5), Cer (32:1), Cer (34:1), Cer (36:1), Cer (38:1), Cer (38:4), Cer (40:2), Cer (40:4), DAG (28:0), DAG (32:0), DAG (32:2), DAG (34:0), DAG (34:3), DAG (34:5), DAG (36:0), DAG (36:1), DAG (36:2), DAG (36:3), DAG (36:8), DAG (38:1), DAG (38:10), DAG (38:2), DAG (38:3), DAG (38:5), DAG (40:1), DAG (40:2), DAG (40:5), DH-LTB4 (20:3), FA (16:3), FA (19:1), GA2 (30:0), GA2 (33:2), GA2 (35:2), GA2 (37:2), GM3 (41:1), HexCer (32:1), HexDHCer (34:0), LacCer (30:0), LacCer (30:1), LacCer (32:2), LysoPA (16:2), LysoPA (16:3), LysoPA (18:1), LysoPA (22:0), LysoPA (22:1), LysoPC (16:0), LysoPC (18:0), LysoPC (18:1), LysoPC (18:4), LysoPC (20:4), LysoPC (20:5), LysoPC (26:6), LysoPC-pmg (12:0), LysoPC-pmg (18:3), LysoPC-pmg (24:4), LysoPC-pmg (26:0), LysoPE (10:1), LysoPE (16:2), LysoPE (18:2), LysoPE-pmg (18:4), LysoPS (24:1), MAG (18:0), MAG (20:3), MAG (24:2), PC (32:0), PC (32:1), PC (34:1), PC (34:2), PC (34:3), PC (34:4), PC (34:6), PC (36:1), PC (36:2), PC (36:3), PC (36:4), PC (36:5), PC (36:6), PC (36:9), PC (38:2), PC (38:3), PC (38:4), PC (38:5), PC (38:6), PC (38:7), PC (38:8), PC (38:9), PC (40:5), PC (40:6), PC (40:7), PC (40:8), PC (40:9), PC (44:12), PC-pmg (30:1), PC-pmg (36:4), PC-pmg (38:5), PC-pmg (38:7), PC-pmg (40:11), PC-pmg (42:1), PE (34:7), PE (36:5), PE (36:7), PE (38:2), PE (38:3), PE (38:4), PE (38:5), PE (38:7), PE (40:4), PE (40:9), PE (42:12), PE (44:11), PE-pmg (28:2), PE-pmg (30:3), PE-pmg (34:6), PE-pmg (34:8), PE-pmg (36:5), PE-pmg (36:6), PE-pmg (40:7), PE-pmg (40:8), PE-pmg (42:10), PE-pmg (42:12), PE-pmg (42:4), PE-pmg (42:7), PE-pmg (42:8), PE-pmg (42:9), PE-pmg (44:10), PE-pmg (44:11), PE-pmg (44:12), PE-pmg (44:7), PE-pmg (44:8), PE-pmg (44:9), PGA1 (20:1), PGB1 (20:1), SM (34:1), SM (34:2), SM (36:1), SM (38:1), SM (40:1), SM (40:2), SM (42:1), SM (42:2), SM (42:3), Sphingosine (18:0), TAG (44:1), TAG (44:3), TAG (46:0), TAG (46:1), TAG (46:2), TAG (46:3), TAG (46:4), TAG (48:0), TAG (48:1), TAG (48:2), TAG (48:3), TAG (48:4), TAG (48:5), TAG (49:1), TAG (49:2), TAG (49:3), TAG (50:0), TAG (50:1), TAG (50:2), TAG (50:3), TAG (50:4), TAG (50:5), TAG (50:6), TAG (51:2), TAG (51:4), TAG (52:2), TAG (52:3), TAG (52:4), TAG (52:5), TAG (52:6), TAG (52:7), TAG (53:4), TAG (54:2), TAG (54:3), TAG (54:4), TAG (54:5), TAG (54:6), TAG (54:7), TAG (54:8), TAG (55:5), TAG (55:6), TAG (55:7), TAG (56:4), TAG (56:5), TAG (56:6), TAG (56:7), TAG (56:8), TAG (56:9), TAG (58:10), TAG (58:6), TAG (58:8), TAG (58:9), TAG (60:12), and TH-12-keto-LTB4(20:2).

8. The method of any one of claims 1 to 4, wherein the at least one cancer type comprises lung cancer and one or more lipids in the lipid set are further selected from the group consisting of LysoPA (22:0), PE-pmg (42:9), FA (16:3), FA (19:1), CE (18:2), Cer (36:1), Cer (38:4), PC (38:5), Cer (38:1), and TAG (44:3).

9. The method of any one of claims 1 to 4, wherein the at least one cancer type comprises lung cancer and one or more lipids in the lipid set are further selected from the group consisting of TAG (44:3), PC (36:5), PC (38:5), Cer (38:4), PE-pmg (42:9), PC (38:7), LysoPA (22:0), Cer (38:1), Cer (34:1), Cer (36:1), PC (40:7), TAG (54:5), TAG (54:6), CE (18:2), PC (36:4), FA (16:3), PE-pmg (44:11), TAG (52:5), Cer (40:4), CE (20:5), PC (38:6), TAG (50:2), MAG (18:0), FA (19:1), TAG (52:2), LysoPA (22:1), MAG (24:2), TAG (54:7), TAG (50:3), TAG (50:1), DAG (36:3), PC (34:1), TAG (52:6), PE-pmg (44:12), CE (20:4), PE (44:11), PC (40:8), TAG (56:9), PE-pmg (34:6), PE (36:7), PE (36:5), TAG (56:7), TAG (56:8), DAG (34:3), TAG (56:6), TAG (52:3), TAG (54:3), TAG (56:5), TAG (54:8), PC (34:6), PC (40:6), DAG (36:0), LysoPE (10:1), DAG (40:5), Cer (32:1), TAG (50:5), TAG (50:4), PE-pmg (36:6), TAG (46:3), and PE (38:5).

10. The method of any one of claims 1 to 4, wherein the at least one cancer type comprises lung cancer and one or more lipids in the lipid set are further selected from the group consisting of TAG (44:3), PC (36:5), PC (38:5), Cer (38:4), PE-pmg (42:9), PC (38:7), LysoPA (22:0), Cer (38:1), Cer (34:1), and Cer (36:1).

11. The method of any one of claims 1 to 4, wherein the at least one cancer type comprises breast cancer and one or more lipids in the lipid set are further selected from the group consisting of LysoPA (22:1), PE-pmg (42:9), CE (20:5), TAG (52:3), LysoPA (22:0), PC (36:3), PC (36:4), PC (36:2), PC (34:2), and PC (34:1).

12. The method of any one of claims 1 to 4, wherein the at least one cancer type comprises breast cancer and one or more lipids in the lipid set are further selected from the group consisting of PC (34:2), PC (34:1), PC (36:2), PC (36:4), PC (36:3), PC (38:4), LysoPA (22:1), PE-pmg (42:9), LysoPA (22:0), CE (20:5), Cer (36:1), CE (18:2), DAG (34:0), SM (34:1), DAG (32:0), PE-pmg (40:8), PC (38:3), DAG (36:0), PC (36:1), TAG (54:5), TAG (54:6), PE-pmg (44:11), PE-pmg (42:8), TAG (52:2), SM (42:2), PC (38:6), TAG (54:7), PC (40:6), PC (40:7), LysoPC (16:0), FA (16:3), TAG (52:5), TAG (44:3), SM (40:1), PE-pmg (42:10), PE-pmg (40:7), SM (36:1), PE (38:2), PC (34:3), PC (36:5), PC (32:0), PC (32:1), PC (36:9), SM (42:3), PC-pmg (36:4), PC-pmg (38:5), PC (40:9), TAG (54:3), PE-pmg (44:12), FA (19:1), TAG (50:3), PC (34:6), GA2 (35:2), TAG (58:9), PE-pmg (42:7), and LysoPC (18:0).

13. The method of any one of claims 1 to 4, wherein the at least one cancer type comprises breast cancer and one or more lipids in the lipid set are further selected from the group consisting of PC (34:2), PC (34:1), PC (36:2), PC (36:4), PC (36:3), PC (38:4), LysoPA (22:1), PE-pmg (42:9), LysoPA (22:0), and CE (20:5).

14. The method of any one of claims 1 to 4, wherein the at least one cancer type comprises lung cancer and breast cancer, and one or more lipids in the lipid set are further selected from the group consisting of LysoPA (22:1), PC (36:5), TAG (52:3), PC (38:5), CE (20:5), TAG (50:2), PC (34:2), CE (18:2), and PC (34:1).

15. The method of any one of claims 1 to 4, wherein the at least one cancer type comprises lung cancer and breast cancer, and one or more lipids in the lipid set are further selected from the group consisting of PC (34:2), PC (36:2), TAG (44:3), CE (18:2), PC (34:1), LysoPA (22:1), PC (36:5), Cer (36:1), CE (20:5), PC (36:3), PC (38:4), PC (36:4), Cer (38:4), PC (38:5), PC (38:7), Cer (38:1), TAG (50:2), Cer (34:1), SM (34:1), Cer (40:4), MAG (18:0), MAG (24:2), PC (38:3), PE-pmg (40:8), PE-pmg (42:8), TAG (50:1), DAG (32:0), PC (36:1), DAG (34:0), LysoPC (16:0), PE-pmg (34:6), DAG (36:3), PC (36:9), PE (36:5), TAG (52:6), FA (19:1), PE-pmg (44:11), PE (44:11), TAG (48:2), SM (42:2), PE-pmg (42:10), PE (36:7), PE-pmg (40:7), PE-pmg (36:6), PE (38:5), PC (32:0), PE (38:2), GA2 (35:2), DAG (34:3), PE-pmg (44:12), MAG (16:0), PC (32:1), LysoPE (10:1), SM (36:1), TAG (56:7), and PE-pmg (42:9).

16. The method of any one of claims 1 to 4, wherein the at least one cancer type comprises lung cancer and breast cancer, and one or more lipids in the lipid set are further selected from the group consisting of PC (34:2), PC (36:2), TAG (44:3), CE (18:2), PC (34:1), LysoPA (22:1), PC (36:5), Cer (36:1), CE (20:5), and PC (36:3).

17. The method of any preceding claim, wherein the lipid amounts are determined using mass spectrometry, such as a Fourier transform ion cyclotron resonance mass analyzer.

18. The method of any preceding claim, wherein the sample is a treatment of a bodily fluid and the treatment comprises one or more extractions using one or more solutions comprising acetonitrile, water, chloroform, methanol, butylated hydroxytoluene, trichloroacetic acid, or combinations thereof.

19. The method of any preceding claim, wherein the predictive model comprises one or more dimension reduction methods.

20. The method of any preceding claim, wherein the predictive model comprises one or more methods selected from the group consisting of principal component analysis (PCA), soft independent modeling of class analogy (SIMCA), partial least squares discriminant analysis (PLS-DA), and orthogonal partial least squares discriminant analysis (OPLS-DA).

21. The method of any preceding claim, wherein the animal is selected from the group consisting of human, dog, cat, horse, cow, pig, sheep, chicken, turkey, mouse, and rat.

22. The method of any preceding claim, wherein the at least one cancer type is lung or breast cancer.

## Patentansprüche

1. Verfahren für die Bestimmung der Gegenwart oder Abwesenheit von mindestens einer Krebsart bei einem Tier, das Folgendes umfasst:
- Bestimmen von Lipidmengen von Lipiden in einer Lipidreihe in einer Probe von dem Tier und
- Bestimmen der Gegenwart oder Abwesenheit von mindestens einer Krebsart bei dem Tier mit einem Vorhersagemodell;
wobei
- die Lipidmengen von Lipiden in der Lipidreihe eine Eingabe des Vorhersagemodells umfassen;
- die Probe eine Körperflüssigkeit oder Behandlung davon umfasst;
- die mindestens eine Krebsart aus der Gruppe ausgewählt ist, die aus Folgenden besteht: Karzinomen, Sarkomen, Blutkrebsarten, neurologischen Malignomen, Schilddrüsenkrebs, Neuroblastom, Melanom, Nierenzellkarzinom, Leberzellkarzinom, Brustkrebs, Kolonkrebs, Lungenkrebs, Bauchspeicheldrüsenkrebs, Hirnkrebs, Prostatakrebs, chronischer lymphozytischer Leukämie, akuter lymphoblastischer Leukämie, Rhabdomyosarkom, Glioblastoma multiforme, Meningeom, Blasenkrebs, Magenkrebs, Gliom, Mundkrebs, Nasopharynxkarzinom, Nierenkrebs, Rektumkarzinom, Lymphknotenkrebs, Knochenmarkkrebs, Magenkrebs, Gebärmutterkrebs, Leukämie, Basalzellkarzinom, Krebsarten, die mit Epithelzellen verbunden sind, Krebsarten, die die Regulation oder Aktivität von Pyruvatcarboxylase verändern können, und Tumoren, die mit jedweder der vorgenannten Krebsarten verbunden sind;
- die Lipidreihe ein Lipid aus der Klasse von BMP umfasst, bevorzugt BMP (30:1), BMP (32:1), BMP (34:1), BMP (35:4), BMP (36:3), BMP (37:1), BMP (37:7), BMP (38:1), BMP (38:2), BMP (38:4), BMP (39:1), BMP (39:4), BMP (40:1), BMP (40:2), BMP (40:3), BMP (40:4), BMP (40:7), BMP (42:10), BMP (42:2), BMP (42:5) oder BMP (44:8), und
- das Vorhersagemodell eines oder mehrere von Folgenden umfasst: Dimensionsreduktionsverfahren, Clustering-Verfahren, maschinelles Lernverfahren, Hauptkomponentenanalyse, Soft Independent Modeling of Class Analogy, Partial Least Squares Regression, Orthogonal Least Squares Regression, Partial Least Squares Discriminant Analysis, Orthogonal Partial Least Squares Discriminant Analysis, Mittelwertzentrierung, Medianzentrierung, Pareto Scaling, Unit Variance Scaling, orthogonale Signalkorrektur, Integration, Differentiation, Kreuzvalidierung oder Receiver-Operating-Characteristic-Kurven.

2. Verfahren nach Anspruch 1, wobei die Körperflüssigkeit aus der Gruppe ausgewählt ist, die aus Folgenden besteht: Plasma Erbrochenes, Cerumen, Magensaft, Muttermilch, Schleim, Speichel, Talg, Sperma, Schweiß, Tränen, Vaginalsekret, Blutserum, Kammerwasser, Glaskörperflüssigkeit, Endolymphe, Perilymphe, Peritonealflüssigkeit, Pleuraflüssigkeit, Liquor, Blut, Plasma, Brustwarzen-Aspiratflüssigkeit, Urin, Stuhl und bronchoalveolärer Lavageflüssigkeit.

3. Verfahren nach Anspruch 1 oder 2, wobei die Probe eine Lipid-Mikrovesikel-Fraktion umfasst.

4. Verfahren nach Anspruch 1, 2 oder 3, wobei die Lipidreihe mindestens 10, mindestens 50, mindestens 100, mindestens 200 oder nicht mehr als 100 000 Lipide umfasst.

5. Verfahren nach einem vorstehenden Anspruch, wobei die Lipidreihe weiter ein oder mehrere Lipide umfasst, die aus der einen oder den mehreren Klassen von Lipiden ausgewählt sind, die aus der Gruppe ausgewählt sind, die aus Folgenden besteht: CE, Cer, DAG, DH-LTB4, FA, GA2, GM3, HexCer, HexDHCer, LacCer, LysoPA, LysoPC, LysoPC-pmg, LysoPE, LysoPE-pmg, LysoPS, MAG, PC, PC-pmg, PE, PE-pmg, PGA1, PGB1, SM, Sphingosin, TAG und TH-12-keto-LTB4.

6. Verfahren nach einem der Ansprüche 1 bis 4, wobei die Lipidreihe weiter ein oder mehrere Lipide umfasst, die aus der einen oder den mehreren Klassen von Lipiden ausgewählt sind, die aus der Gruppe ausgewählt sind, die aus Folgenden besteht: FA, MAG, DAG, TAG, PI, PE, PS, PI, PG, PA, LysoPC, LysoPE, LysoPS, LysoPI, LysoPG, LysoPA, LysoPC, LysoPE, SM, Cer, Cer-P, HexCer, GA1, GA2, GD1, GD2, GM1, GM2, GM3, GT1 und CE.

7. Verfahren nach einem der Ansprüche 1 bis 4, wobei ein oder mehrere Lipide in der Lipidreihe weiter aus der Gruppe ausgewählt sind, die aus Folgenden besteht: CE (16:2), CE (18:2), CE (18:3), CE (18:4), CE (20:2), CE (20:4), CE (20:5), Cer (32:1), Cer (34:1), Cer (36:1), Cer (38:1), Cer (38:4), Cer (40:2), Cer (40:4), DAG (28:0), DAG (32:0), DAG (32:2), DAG (34:0), DAG (34:3), DAG (34:5), DAG (36:0), DAG (36:1), DAG (36:2), DAG (36:3), DAG (36:8), DAG (38:1), DAG (38:10), DAG (38:2), DAG (38:3), DAG (38:5), DAG (40:1), DAG (40:2), DAG (40:5), DH-LTB4 (20:3), FA (16:3), FA (19:1), GA2 (30:0), GA2 (33:2), GA2 (35:2), GA2 (37:2), GM3 (41:1), HexCer (32:1), HexDHCer (34:0), LacCer (30:0), LacCer (30:1), LacCer (32:2), LysoPA (16:2), LysoPA (16:3), LysoPA (18:1), LysoPA (22:0), LysoPA (22:1), LysoPC (16:0), LysoPC (18:0), LysoPC (18:1), LysoPC (18:4), LysoPC (20:4), LysoPC (20:5), LysoPC (26:6), LysoPC-pmg (12:0), LysoPC-pmg (18:3), LysoPC-pmg (24:4), LysoPC-pmg (26:0), LysoPE (10:1), LysoPE (16:2), LysoPE (18:2), LysoPE-pmg (18:4), LysoPS (24:1), MAG (18:0), MAG (20:3), MAG (24:2), PC (32:0), PC (32:1), PC (34:1), PC (34:2), PC (34:3), PC (34:4), PC (34:6), PC (36:1), PC (36:2), PC (36:3), PC (36:4), PC (36:5), PC (36:6), PC (36:9), PC (38:2), PC (38:3), PC (38:4), PC (38:5), PC (38:6), PC (38:7), PC (38:8), PC (38:9), PC (40:5), PC (40:6), PC (40:7), PC (40:8), PC (40:9), PC (44:12), PC-pmg (30:1), PC-pmg (36:4), PC-pmg (38:5), PC-pmg (38:7), PC-pmg (40:11), PC-pmg (42:1), PE (34:7), PE (36:5), PE (36:7), PE (38:2), PE (38:3), PE (38:4), PE (38:5), PE (38:7), PE (40:4), PE (40:9), PE (42:12), PE (44:11), PE-pmg (28:2), PE-pmg (30:3), PE-pmg (34:6), PE-pmg (34:8), PE-pmg (36:5), PE-pmg (36:6), PE-pmg (40:7), PE-pmg (40:8), PE-pmg (42:10), PE-pmg (42:12), PE-pmg (42:4), PE-pmg (42:7), PE-pmg (42:8), PE-pmg (42:9), PE-pmg (44:10), PE-pmg (44:11), PE-pmg (44:12), PE-pmg (44:7), PE-pmg (44:8), PE-pmg (44:9), PGA1 (20:1), PGB1 (20:1), SM (34:1), SM (34:2), SM (36:1), SM (38:1), SM (40:1), SM (40:2), SM (42:1), SM (42:2), SM (42:3), Sphingosin (18:0), TAG (44:1), TAG (44:3), TAG (46:0), TAG (46:1), TAG (46:2), TAG (46:3), TAG (46:4), TAG (48:0), TAG (48:1), TAG (48:2), TAG (48:3), TAG (48:4), TAG (48:5), TAG (49:1), TAG (49:2), TAG (49:3), TAG (50:0), TAG (50:1), TAG (50:2), TAG (50:3), TAG (50:4), TAG (50:5), TAG (50:6), TAG (51:2), TAG (51:4), TAG (52:2), TAG (52:3), TAG (52:4), TAG (52:5), TAG (52:6), TAG (52:7), TAG (53:4), TAG (54:2), TAG (54:3), TAG (54:4), TAG (54:5), TAG (54:6), TAG (54:7), TAG (54:8), TAG (55:5), TAG (55:6), TAG (55:7), TAG (56:4), TAG (56:5), TAG (56:6), TAG (56:7), TAG (56:8), TAG (56:9), TAG (58:10), TAG (58:6), TAG (58:8), TAG (58:9), TAG (60:12) und TH-12-keto-LTB4 (20:2).

8. Verfahren nach einem der Ansprüche 1 bis 4, wobei die mindestens eine Krebsart Lungenkrebs umfasst und ein oder mehrere Lipide in der Lipidreihe weiter aus der Gruppe ausgewählt sind, die aus Folgenden besteht: LysoPA (22:0), PE-pmg (42:9), FA (16:3), FA (19:1), CE (18:2), Cer (36:1), Cer (38:4), PC (38:5), Cer (38:1) und TAG (44:3).

9. Verfahren nach einem der Ansprüche 1 bis 4, wobei die mindestens eine Krebsart Lungenkrebs umfasst und ein oder mehrere Lipide in der Lipidreihe weiter aus der Gruppe ausgewählt sind, die aus Folgenden besteht: TAG (44:3), PC (36:5), PC (38:5), Cer (38:4), PE-pmg (42:9), PC (38:7), LysoPA (22:0), Cer (38:1), Cer (34:1), Cer (36:1), PC (40:7), TAG (54:5), TAG (54:6), CE (18:2), PC (36:4), FA (16:3), PE-pmg (44:11), TAG (52:5), Cer (40:4), CE (20:5), PC (38:6), TAG (50:2), MAG (18:0), FA (19:1), TAG (52:2), LysoPA (22:1), MAG (24:2), TAG (54:7), TAG (50:3), TAG (50:1), DAG (36:3), PC (34:1), TAG (52:6), PE-pmg (44:12), CE (20:4), PE (44:11), PC (40:8), TAG (56:9), PE-pmg (34:6), PE (36:7), PE (36:5), TAG (56:7), TAG (56:8), DAG (34:3), TAG (56:6), TAG (52:3), TAG (54:3), TAG (56:5), TAG (54:8), PC (34:6), PC (40:6), DAG (36:0), LysoPE (10:1), DAG (40:5), Cer (32:1), TAG (50:5), TAG (50:4), PE-pmg (36:6), TAG (46:3) und PE (38:5).

10. Verfahren nach einem der Ansprüche 1 bis 4, wobei die mindestens eine Krebsart Lungenkrebs umfasst und ein oder mehrere Lipide in der Lipidreihe weiter aus der Gruppe ausgewählt sind, die aus Folgenden besteht: TAG (44:3), PC (36:5), PC (38:5), Cer (38:4), PE-pmg (42:9), PC (38:7), LysoPA (22:0), Cer (38:1), Cer (34:1) und Cer (36:1).

11. Verfahren nach einem der Ansprüche 1 bis 4, wobei die mindestens eine Krebsart Brustkrebs umfasst und ein oder mehrere Lipide in der Lipidreihe weiter aus der Gruppe ausgewählt sind, die aus Folgenden besteht: LysoPA (22:1), PE-pmg (42:9), CE (20:5), TAG (52:3), LysoPA (22:0), PC (36:3), PC (36:4), PC (36:2), PC (34:2) und PC (34:1).

12. Verfahren nach einem der Ansprüche 1 bis 4, wobei die mindestens eine Krebsart Brustkrebs umfasst und ein oder mehrere Lipide in der Lipidreihe weiter aus der Gruppe ausgewählt sind, die aus Folgenden besteht: PC (34:2), PC (34:1), PC (36:2), PC (36:4), PC (36:3), PC (38:4), LysoPA (22:1), PE-pmg (42:9), LysoPA (22:0), CE (20:5), Cer (36:1), CE (18:2), DAG (34:0), SM (34:1), DAG (32:0), PE-pmg (40:8), PC (38:3), DAG (36:0), PC (36:1), TAG (54:5), TAG (54:6), PE-pmg (44:11), PE-pmg (42:8), TAG (52:2), SM (42:2), PC (38:6), TAG (54:7), PC (40:6), PC (40:7), LysoPC (16:0), FA (16:3), TAG (52:5), TAG (44:3), SM (40:1), PE-pmg (42:10), PE-pmg (40:7), SM (36:1), PE (38:2), PC (34:3), PC (36:5), PC (32:0), PC (32:1), PC (36:9), SM (42:3), PC-pmg (36:4), PC-pmg (38:5), PC (40:9), TAG (54:3), PE-pmg (44:12), FA (19:1), TAG (50:3), PC (34:6), GA2 (35:2), TAG (58:9), PE-pmg (42:7) und LysoPC (18:0).

13. Verfahren nach einem der Ansprüche 1 bis 4, wobei die mindestens eine Krebsart Brustkrebs umfasst und ein oder mehrere Lipide in der Lipidreihe weiter aus der Gruppe ausgewählt sind, die aus Folgenden besteht: PC (34:2), PC (34:1), PC (36:2), PC (36:4), PC (36:3), PC (38:4), LysoPA (22:1), PE-pmg (42:9), LysoPA (22:0) und CE (20:5).

14. Verfahren nach einem der Ansprüche 1 bis 4, wobei die mindestens eine Krebsart Lungenkrebs und Brustkrebs umfasst und ein oder mehrere Lipide in der Lipidreihe weiter aus der Gruppe ausgewählt sind, die aus Folgenden besteht: LysoPA (22:1), PC (36:5), TAG (52:3), PC (38:5), CE (20:5), TAG (50:2), PC (34:2), CE (18:2) und PC (34:1).

15. Verfahren nach einem der Ansprüche 1 bis 4, wobei die mindestens eine Krebsart Lungenkrebs und Brustkrebs umfasst und ein oder mehrere Lipide in der Lipidreihe weiter aus der Gruppe ausgewählt sind, die aus Folgenden besteht: PC (34:2), PC (36:2), TAG (44:3), CE (18:2), PC (34:1), LysoPA (22:1), PC (36:5), Cer (36:1), CE (20:5), PC (36:3), PC (38:4), PC (36:4), Cer (38:4), PC (38:5), PC (38:7), Cer (38:1), TAG (50:2), Cer (34:1), SM (34:1), Cer (40:4), MAG (18:0), MAG (24:2), PC (38:3), PE-pmg (40:8), PE-pmg (42:8), TAG (50:1), DAG (32:0), PC (36:1), DAG (34:0), LysoPC (16:0), PE-pmg (34:6), DAG (36:3), PC (36:9), PE (36:5), TAG (52:6), FA (19:1), PE-pmg (44:11), PE (44:11), TAG (48:2), SM (42:2), PE-pmg (42:10), PE (36:7), PE-pmg (40:7), PE-pmg (36:6), PE (38:5), PC (32:0), PE (38:2), GA2 (35:2), DAG (34:3), PE-pmg (44:12), MAG (16:0), PC (32:1), LysoPE (10:1), SM (36:1), TAG (56:7) und PE-pmg (42:9).

16. Verfahren nach einem der Ansprüche 1 bis 4, wobei die mindestens eine Krebsart Lungenkrebs und Brustkrebs umfasst und ein oder mehrere Lipide in der Lipidreihe weiter aus der Gruppe ausgewählt sind, die aus Folgenden besteht: PC (34:2), PC (36:2), TAG (44:3), CE (18:2), PC (34:1), LysoPA (22:1), PC (36:5), Cer (36:1), CE (20:5) und PC (36:3).

17. Verfahren nach einem vorstehenden Anspruch, wobei die Lipidmengen unter Verwendung von Massenspektrometrie, wie zum Beispiel einem Fourier-Transform-Ionen-Cyclotron-Resonanz-Massenanalysator, bestimmt werden.

18. Verfahren nach einem vorstehenden Anspruch, wobei die Probe eine Behandlung einer Körperflüssigkeit ist und die Behandlung eine oder mehrere Extraktionen unter Verwendung von einer oder mehreren Lösungen, die Acetonitril, Wasser, Chloroform, Methanol, butyliertes Hydroxytoluol, Trichloressigsäure oder Kombinationen davon umfassen, umfasst.

19. Verfahren nach einem vorstehenden Anspruch, wobei das Vorhersagemodell ein oder mehrere Dimensionsreduktionsverfahren umfasst.

20. Verfahren nach einem vorstehenden Anspruch, wobei das Vorhersagemodell ein oder mehrere Verfahren umfasst, die aus der Gruppe ausgewählt sind, die aus Folgenden besteht: Hauptkomponentenanalyse (PCA), Soft Independent Modeling of Class Analogy (SIMCA), Partial Least Squares Discriminant Analysis (PLS-DA) und Orthogonal Partial Least Squares Discriminant Analysis (OPLS-DA).

21. Verfahren nach einem vorstehenden Anspruch, wobei das Tier aus der Gruppe ausgewählt ist, die aus Folgenden besteht: Mensch, Hund, Katze, Pferd, Rind, Schwein, Schaf, Huhn, Truthahn, Maus und Ratte.

22. Verfahren nach einem vorstehenden Anspruch, wobei die mindestens eine Krebsart Lungen- oder Brustkrebs ist.

## Revendications

1. Un procédé pour la détermination de la présence ou l'absence d'au moins un type de cancer chez un animal comprenant
- la détermination de quantités de lipides des lipides dans un ensemble de lipides dans un échantillon provenant de l'animal, et
- la détermination de la présence ou l'absence d'au moins un type de cancer chez l'animal avec un modèle prédictif ;
dans lequel
- les quantités de lipides des lipides dans l'ensemble de lipides comprennent une entrée du modèle prédictif,
- l'échantillon comprend un fluide corporel ou un traitement de celui-ci ;
- le au moins un type de cancer est sélectionné dans le groupe constitué des carcinomes, des sarcomes, des cancers hématologiques, des malignités neurologiques, du cancer de la thyroïde, du neuroblastome, du mélanome, du carcinome à cellules rénales, du carcinome hépatocellulaire, du cancer du sein, du cancer du côlon, du cancer du poumon, du cancer du pancréas, du cancer du cerveau, du cancer de la prostate, de la leucémie lymphoïde chronique, de la leucémie lymphoblastique aiguë, du rhabdomyosarcome, du glioblastome multiforme, du méningiome, du cancer de la vessie, du cancer gastrique, du gliome, du cancer buccal, du carcinome nasopharyngé, du cancer du rein, du cancer rectal, du cancer des ganglions lymphatiques, du cancer de la moelle épinière, du cancer de l'estomac, du cancer de l'utérus, de la leucémie, du carcinome des cellules basales, des cancers liés aux cellules épithéliales, des cancers qui peuvent modifier la régulation ou l'activité de la pyruvate carboxylase, et des tumeurs associées à l'un quelconque des types de cancer susmentionnés ;
- l'ensemble de lipides comprend un lipide de la classe des BMP, de préférence BMP (30:1), BMP (32:1), BMP (34:1), BMP (35:4), BMP (36:3), BMP (37:1), BMP (37:7), BMP (38:1), BMP (38:2), BMP (38:4), BMP (39:1), BMP (39:4), BMP (40:1), BMP (40:2), BMP (40:3), BMP (40:4), BMP (40:7), BMP (42:10), BMP (42:2), BMP (42:5), ou BMP (44:8), et
- le modèle prédictif comprend un ou plusieurs procédés parmi le procédé de réduction de la dimension, le procédé de regroupement, le procédé d'apprentissage machine, l'analyse en composantes principales, la modélisation indépendante souple des analogies de classes, la régression par les moindres carrés partiels, la régression par les moindres carrés orthogonaux, l'analyse discriminante par les moindres carrés partiels, l'analyse discriminante par les moindres carrés partiels orthogonaux, le centrage par rapport à la moyenne, le centrage médian, la mise à l'échelle de Pareto, la mise à l'échelle de la variance unitaire, la correction des signaux orthogonaux, l'intégration, la différentiation, la validation croisée, ou les courbes caractéristiques de fonctionnement du récepteur.

2. Procédé de la revendication 1, dans lequel le fluide corporel est sélectionné dans le groupe constitué de vomissures de plasma, de cérumen, de suc gastrique, de lait maternel, de mucus, de salive, de sébum, de sperme, de sueur, de larmes, de sécrétions vaginales, de sérum sanguin, d'humeur aqueuse, d'humeur vitrée, d'endolymphe, de périlymphe, de fluide péritonéal, de fluide pleural, de fluide cérébrospinal, de sang, de plasma, de fluide obtenu par aspiration mammaire, d'urine, de selles, et de fluide de lavage broncho-alvéolaire.

3. Procédé de la revendication 1 ou 2, dans lequel l'échantillon comprend un fragment de microvésicule lipidique.

4. Procédé de la revendication 1, 2 ou 3, dans lequel l'ensemble de lipides comprend au moins 10, au moins 50, au moins 100, au moins 200 ou pas plus de 100 000 lipides.

5. Procédé de l'une quelconque des revendications précédentes, dans lequel l'ensemble de lipides comprend en outre un ou plusieurs lipides sélectionnés parmi les une ou plusieurs classes de lipides sélectionnées dans le groupe constitué des CE, Cer, DAG, DH-LTB4, FA, GA2, GM3, HexCer, HexDHCer, LacCer, LysoPA, LysoPC, LysoPC-pmg, LysoPE, LysoPE-pmg, LysoPS, MAG, PC, PC-pmg, PE, PE-pmg, PGA1, PGB1, SM, Sphingosine, TAG, et TH-12-céto-LTB4.

6. Procédé de l'une quelconque des revendications 1 à 4, dans lequel l'ensemble de lipides comprend en outre un ou plusieurs lipides sélectionnés parmi les une ou plusieurs classes de lipides sélectionnées dans le groupe constitué de FA, MAG, DAG, TAG, PI, PE, PS, PI, PG, PA, LysoPC, LysoPE, LysoPS, LysoPI, LysoPG, LysoPA, LysoPC, LysoPE, SM, Cer, Cer-P, HexCer, GA1, GA2, GD1, GD2, GM1, GM2, GM3, GT1, et CE.

7. Procédé de l'une quelconque des revendications 1 à 4, dans lequel un ou plusieurs lipides dans l'ensemble de lipides sont sélectionnés en outre parmi le groupe constitué de CE (16:2), CE (18:2), CE (18:3), CE (18:4), CE (20:2), CE (20:4), CE (20:5), Cer (32:1), Cer (34:1), Cer (36:1), Cer (38:1), Cer (38:4), Cer (40:2), Cer (40:4), DAG (28:0), DAG (32:0), DAG (32:2), DAG (34:0), DAG (34:3), DAG (34:5), DAG (36:0), DAG (36:1), DAG (36:2), DAG (36:3), DAG (36:8), DAG (38:1), DAG (38:10), DAG (38:2), DAG (38:3), DAG (38:5), DAG (40:1), DAG (40:2), DAG (40:5), DH-LTB4 (20:3), FA (16:3), FA (19:1), GA2 (30:0), GA2 (33:2), GA2 (35:2), GA2 (37:2), GM3 (41:1), HexCer (32:1), HexDHCer (34:0), LacCer (30:0), LacCer (30:1), LacCer (32:2), LysoPA (16:2), LysoPA (16:3), LysoPA (18:1), LysoPA (22:0), LysoPA (22:1), LysoPC (16:0), LysoPC (18:0), LysoPC (18:1), LysoPC (18:4), LysoPC (20:4), LysoPC (20:5), LysoPC (26:6), LysoPC-pmg (12:0), LysoPC-pmg (18:3), LysoPC-pmg (24:4), LysoPC-pmg (26:0), LysoPE (10:1), LysoPE (16:2), LysoPE (18:2), LysoPE-pmg (18:4), LysoPS (24:1), MAG (18:0), MAG (20:3), MAG (24:2), PC (32:0), PC (32:1), PC (34:1), PC (34:2), PC (34:3), PC (34:4), PC (34:6), PC (36:1), PC (36:2), PC (36:3), PC (36:4), PC (36:5), PC (36:6), PC (36:9), PC (38:2), PC (38:3), PC (38:4), PC (38:5), PC (38:6), PC (38:7), PC (38:8), PC (38:9), PC (40:5), PC (40:6), PC (40:7), PC (40:8), PC (40:9), PC (44:12), PC-pmg (30:1), PC-pmg (36:4), PC-pmg (38:5), PC-pmg (38:7), PC-pmg (40:11), PC-pmg (42:1), PE (34:7), PE (36:5), PE (36:7), PE (38:2), PE (38:3), PE (38:4), PE (38:5), PE (38:7), PE (40:4), PE (40:9), PE (42:12), PE (44:11), PE-pmg (28:2), PE-pmg (30:3), PE-pmg (34:6), PE-pmg (34:8), PE-pmg (36:5), PE-pmg (36:6), PE-pmg (40:7), PE-pmg (40:8), PE-pmg (42:10), PE-pmg (42:12), PE-pmg (42:4), PE-pmg (42:7), PE-pmg (42:8), PE-pmg (42:9), PE-pmg (44:10), PE-pmg (44:11), PE-pmg (44:12), PE-pmg (44:7), PE-pmg (44:8), PE-pmg (44:9), PGA1 (20:1), PGB1 (20:1), SM (34:1), SM (34:2), SM (36:1), SM (38:1), SM (40:1), SM (40:2), SM (42:1), SM (42:2), SM (42:3), Sphingosine (18:0), TAG (44:1), TAG (44:3), TAG (46:0), TAG (46:1), TAG (46:2), TAG (46:3), TAG (46:4), TAG (48:0), TAG (48:1), TAG (48:2), TAG (48:3), TAG (48:4), TAG (48:5), TAG (49:1), TAG (49:2), TAG (49:3), TAG (50:0), TAG (50:1), TAG (50:2), TAG (50:3), TAG (50:4), TAG (50:5), TAG (50:6), TAG (51:2), TAG (51:4), TAG (52:2), TAG (52:3), TAG (52:4), TAG (52:5), TAG (52:6), TAG (52:7), TAG (53:4), TAG (54:2), TAG (54:3), TAG (54:4), TAG (54:5), TAG (54:6), TAG (54:7), TAG (54:8), TAG (55:5), TAG (55:6), TAG (55:7), TAG (56:4), TAG (56:5), TAG (56:6), TAG (56:7), TAG (56:8), TAG (56:9), TAG (58:10), TAG (58:6), TAG (58:8), TAG (58:9), TAG (60:12), et TH-12-céto-LTB4 (20:2).

8. Procédé de l'une quelconque des revendications 1 à 4, dans lequel le au moins un type de cancer comprend le cancer du poumon et un ou plusieurs lipides dans l'ensemble de lipides sont sélectionnés en outre dans le groupe constitué de LysoPA (22:0), PE-pmg (42:9), FA (16:3), FA (19:1), CE (18:2), Cer (36:1), Cer (38:4), PC (38:5), Cer (38:1), et TAG (44:3).

9. Procédé de l'une quelconque des revendications 1 à 4, dans lequel le au moins un type de cancer comprend le cancer du poumon et un ou plusieurs lipides dans l'ensemble de lipides sont sélectionnés en outre dans le groupe constitué de TAG (44:3), PC (36:5), PC (38:5), Cer (38:4), PE-pmg (42:9), PC (38:7), LysoPA (22:0), Cer (38:1), Cer (34:1), Cer (36:1), PC (40:7), TAG (54:5), TAG (54:6), CE (18:2), PC (36:4), FA (16:3), PE-pmg (44:11), TAG (52:5), Cer (40:4), CE (20:5), PC (38:6), TAG (50:2), MAG (18:0), FA (19:1), TAG (52:2), LysoPA (22:1), MAG (24:2), TAG (54:7), TAG (50:3), TAG (50:1), DAG (36:3), PC (34:1), TAG (52:6), PE-pmg (44:12), CE (20:4), PE (44:11), PC (40:8), TAG (56:9), PE-pmg (34:6), PE (36:7), PE (36:5), TAG (56:7), TAG (56:8), DAG (34:3), TAG (56:6), TAG (52:3), TAG (54:3), TAG (56:5), TAG (54:8), PC (34:6), PC (40:6), DAG (36:0), LysoPE (10:1), DAG (40:5), Cer (32:1), TAG (50:5), TAG (50:4), PE-pmg (36:6), TAG (46:3), et PE (38:5).

10. Procédé de l'une quelconque des revendications 1 à 4, dans lequel le au moins un type de cancer comprend le cancer du poumon et un ou plusieurs lipides dans l'ensemble de lipides sont sélectionnés en outre dans le groupe constitué de TAG (44:3), PC (36:5), PC (38:5), Cer (38:4), PE-pmg (42:9), PC (38:7), LysoPA (22:0), Cer (38:1), Cer (34:1), et Cer (36:1).

11. Procédé de l'une quelconque des revendications 1 à 4, dans lequel le au moins un type de cancer comprend le cancer du sein et un ou plusieurs lipides dans l'ensemble de lipides sont sélectionnés en outre dans le groupe constitué de LysoPA (22:1), PE-pmg (42:9), CE (20:5), TAG (52:3), LysoPA (22:0), PC (36:3), PC (36:4), PC (36:2), PC (34:2), et PC (34:1).

12. Procédé de l'une quelconque des revendications 1 à 4, dans lequel le au moins un type de cancer comprend le cancer du sein et un ou plusieurs lipides dans l'ensemble de lipides sont sélectionnés en outre dans le groupe constitué de PC (34:2), PC (34:1), PC (36:2), PC (36:4), PC (36:3), PC (38:4), LysoPA (22:1), PE-pmg (42:9), LysoPA (22:0), CE (20:5), Cer (36:1), CE (18:2), DAG (34:0), SM (34:1), DAG (32:0), PE-pmg (40:8), PC (38:3), DAG (36:0), PC (36:1), TAG (54:5), TAG (54:6), PE-pmg (44:11), PE-pmg (42:8), TAG (52:2), SM (42:2), PC (38:6), TAG (54:7), PC (40:6), PC (40:7), LysoPC (16:0), FA (16:3), TAG (52:5), TAG (44:3), SM (40:1), PE-pmg (42:10), PE-pmg (40:7), SM (36:1), PE (38:2), PC (34:3), PC (36:5), PC (32:0), PC (32:1), PC (36:9), SM (42:3), PC-pmg (36:4), PC-pmg (38:5), PC (40:9), TAG (54:3), PE-pmg (44:12), FA (19:1), TAG (50:3), PC (34:6), GA2 (35:2), TAG (58:9), PE-pmg (42:7), et LysoPC (18:0).

13. Procédé de l'une quelconque des revendications 1 à 4, dans lequel le au moins un type de cancer comprend le cancer du sein et un ou plusieurs lipides dans l'ensemble de lipides sont sélectionnés en outre dans le groupe constitué de PC (34:2), PC (34:1), PC (36:2), PC (36:4), PC (36:3), PC (38:4), LysoPA (22:1), PE-pmg (42:9), LysoPA (22:0), et CE (20:5).

14. Procédé de l'une quelconque des revendications 1 à 4, dans lequel le au moins un type de cancer comprend le cancer du poumon et le cancer du sein, et un ou plusieurs lipides dans l'ensemble de lipides sont sélectionnés en outre dans le groupe constitué de LysoPA (22:1), PC (36:5), TAG (52:3), PC (38:5), CE (20:5), TAG (50:2), PC (34:2), CE (18:2), et PC (34:1).

15. Procédé de l'une quelconque des revendications 1 à 4, dans lequel le au moins un type de cancer comprend le cancer du poumon et le cancer du sein, et un ou plusieurs lipides dans l'ensemble de lipides sont sélectionnés en outre dans le groupe constitué de PC (34:2), PC (36:2), TAG (44:3), CE (18:2), PC (34:1), LysoPA (22:1), PC (36:5), Cer (36:1), CE (20:5), PC (36:3), PC (38:4), PC (36:4), Cer (38:4), PC (38:5), PC (38:7), Cer (38:1), TAG (50:2), Cer (34:1), SM (34:1), Cer (40:4), MAG (18:0), MAG (24:2), PC (38:3), PE-pmg (40:8), PE-pmg (42:8), TAG (50:1), DAG (32:0), PC (36:1), DAG (34:0), LysoPC (16:0), PE-pmg (34:6), DAG (36:3), PC (36:9), PE (36:5), TAG (52:6), FA (19:1), PE-pmg (44:11), PE (44:11), TAG (48:2), SM (42:2), PE-pmg (42:10), PE (36:7), PE-pmg (40:7), PE-pmg (36:6), PE (38:5), PC (32:0), PE (38:2), GA2 (35:2), DAG (34:3), PE-pmg (44:12), MAG (16:0), PC (32:1), LysoPE (10:1), SM (36:1), TAG (56:7), et PE-pmg (42:9).

16. Procédé de l'une quelconque des revendications 1 à 4, dans lequel le au moins un type de cancer comprend le cancer du poumon et le cancer du sein, et un ou plusieurs lipides dans l'ensemble de lipides sont sélectionnés en outre dans le groupe constitué de PC (34:2), PC (36:2), TAG (44:3), CE (18:2), PC (34:1), LysoPA (22:1), PC (36:5), Cer (36:1), CE (20:5), et PC (36:3).

17. Procédé de l'une quelconque des revendications précédentes, dans lequel les quantités de lipides sont déterminées en utilisant la spectrométrie de masse, tel qu'un analyseur de masse par résonance cyclotronique ionique à transformée de Fourier.

18. Procédé de l'une quelconque des revendications précédentes, dans lequel l'échantillon est un traitement d'un fluide corporel et le traitement comprend une ou plusieurs extractions en utilisant une ou plusieurs solutions comprenant de l'acétonitrile, de l'eau, du chloroforme, du méthanol, de l'hydroxytoluène butylé, de l'acide trichloroacétique ou des combinaisons de ceux-ci.

19. Procédé de l'une quelconque des revendications précédentes, dans lequel le modèle prédictif comprend un ou plusieurs procédés de réduction de la dimension.

20. Procédé de l'une quelconque des revendications précédentes, dans lequel le modèle prédictif comprend un ou plusieurs procédés sélectionnés dans le groupe constitué de l'analyse en composante principale (PCA), la modélisation indépendante souple des analogies de classes (SIMCA), l'analyse discriminante par les moindres carrés partiels (PLS-DA), et l'analyse discriminante par les moindres carrés partiels orthogonaux (OPLS-DA).

21. Procédé de l'une quelconque des revendications précédentes, dans lequel l'animal est sélectionné dans le groupe constitué de l'humain, du chien, du chat, du cheval, de la vache, du cochon, du mouton, du poulet, de la dinde, de la souris et du rat.

22. Procédé de l'une quelconque des revendications précédentes, dans lequel le au moins un type de cancer est le cancer du poumon ou du sein.
